# EUROPEAN PATENT APPLICATION

(11) **EP 1 889 617 A1**
(43) Date of publication of application: **20.02.2008**
(21) Application number: 06116999.1
(22) Date of filing: 11.07.2006
(51) Int. Cl.: A61K 31/40, C07D 207/323, C07D 207/333, C07D 307/42, C07D 333/16, A61K 31/402, A61K 31/381, A61K 31/341, A61P 35/00

(54) **Triphenyl modified 5-membered heterocycles and their use as anticancer and antiflammatory agents**

(71) Applicant: Freie Universität Berlin, 14195 Berlin (DE)
(72) Inventor: Hoffmann, Ricarda, 13089, Berlin (DE); Gust, Ronald, 14513, Teltow (DE)
(74) Representative: Ziebig, Marlene

(57) **Abstract**

The present invention provides 5-membered heterocycles, preferably pyrroles, furans and thiophenes, with three phenyl moieties wherein at least one of the three phenyl moieties is substituted with halogen or trihalomethyl. The compounds of the invention show antiproliferative effects and inhibitory effects on cyclooxygenases (COX-1 and COX-2). Therefore the invention is also directed to the use of said compounds for the manufacture of a pharmaceutical composition for prevention and treatment of cancer and/or inflammatory diseases. The invention further concerns a method for treating cancer or an inflammatory disease, which comprises the step of administering to a patient suffering from that disease a therapeutically effective amount of at least one of said compounds.

## Description

The present invention provides 5-membered heterocycles, preferably pyrroles, furans and thiophenes, with three phenyl moieties wherein at least one of the three phenyl moieties is substituted with halogen or trihalomethyl. The compounds of the invention show antiproliferative effects and inhibitory effects on cyclooxygenases (COX-1 and COX-2). Therefore the invention is also directed to the use of said compounds for the manufacture of a pharmaceutical composition for prevention and treatment of cancer and/or inflammatory diseases. The invention further concerns a method for treating cancer or an inflammatory disease, which comprises the step of administering to a patient suffering from that disease a therapeutically effective amount of at least one of said compounds.

The cyclooxygenase (EC1.14.99.1) is the key enzyme of the prostaglandin biosynthesis that catalyses the conversion of arachidonic acid to prostaglandins. Two isoforms, COX-1 and COX-2, are known. COX-1 is constitutively expressed in most tissues and produces prostaglandins involved in the maintenance of the gastric mucosa, regulation of renal blood flow, and platelet aggregation. The inducible form, COX-2, is not detected in most normal tissues, it is expressed in inflamed and neoplastic tissues and is induced by proinflammatory and mitogenetic stimuli, such as for instance growth factors.

Well known COX-2 inhibitors used since the late 1990s as antiflammatory agents are for instance indomethacin ([1-(4-Chlorobenzoyl)-5-methoxy-2-methyl-1*H*-indol-3-yl]acetic acid), celecoxib (Celebrex®), rofecoxib (Vioxx®), valdecoxib (Bextra®). However, the market withdrawal of Vioxx® and Bextra® due to adverse cardiovascular side effects clearly shows the need to develop alternative anti-inflammatory agents.

During the past few years, cyclooxygenases were also discussed as new targets for anticancer drugs, since abnormally high levels of COX-2 protein were observed in several types of cancer and precancerous tissue, including breast cancer. COX-2 proteins were found to increase new blood vessel formation and proliferation. A tumor growth inhibition can thus be achieved by blocking COX-2.

Therefore the technical problem underlying the present invention is to provide COX-inhibitors showing cytotoxic properties, that means compounds with anti-inflammatory and anticancer potential. The provision of alternative anti-inflammatory agents with reduced adverse cardiovascular effects was another object the present invention.

The problem of the invention is solved by the provision of the embodiments as defined in the claims of the present invention. It has been found that the compounds of formula I, their mixtures, salts or esters effectively inhibit cyclooxygenases, especially COX-1 and/or COX-2, and exhibit cytotoxic properties which make them suitable for treating inflammatory diseases and cancer. Compounds of formula I with a hydroxy group in at least one of the phenyl moieties, preferably in 4-position of the phenyl group, exhibit not only COX-inhibiting and cytotoxic properties, they additionally show estrogenic effects.

Briefly, the present invention provides triphenyl modified 5-membered heterocycles of the general formula I 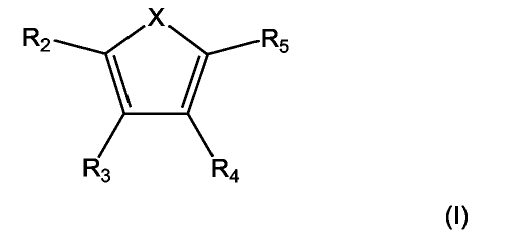 wherein
- X: represents NR¹, O or S
- R¹ to R⁵: independently from each other represent H, alkyl, unsubstituted phenyl or phenyl substituted with hydroxy, alkoxy, alkyl, trihalomethyl and/or halogen, wherein at least one of the three phenyl moieties is substituted with halogen or trihalomethyl.

Compounds 2,3-diphenyl-5-(o-bromophenyl)pyrrole, 2,3-diphenyl-5-(m-iodophenyl)pyrrole, 2-(p-methoxyphenyl-3-(m-ethoxyphenyl)-5-(p-fluorophenyl) pyrrole and 2-(p-fluorophenyl)-3-(m-butylphenyl)-5-(p-trifluoromethyl-phenyl) pyrrole which are described in US 3,531,497 are excluded from the compound claims. But US 3,531,497 does not disclose a biological activity of these intermediate substances.

In a preferred embodiment of the invention at least one of the three phenyl moieties is substituted with halogen and alkoxy, halogen and hydroxy, trihalomethyl and alkoxy, trihalomethyl and hydroxy or halogen and alkyl. It is especially preferred that two or three phenyl moieties are substituted with alkoxy and/or hydroxy. It is also especially preferred that two or three phenyl moieties are substituted with halogen and/or trihalomethyl.

In one preferred aspect of the invention the halogen or trihalomethyl substituent is located in 2-position of the phenyl moiety/-ies, while the hydroxy, alkoxy or alkyl group is located in 4-position of the phenyl moiety. It is also possible that the halogen or trihalomethyl substituent is located at 3-position of the phenyl moiety/-ies and the hydroxy, alkoxy or alky group is located at 5-position. Compounds with halogen or trihalomethyl in 4-position of the phenyl moiety and hydroxy, alkoxy or alkyl in 2-position of the phenyl moiety are also encompassed from the invention.

In a further embodiment of the invention the two non-phenyl moieties of R¹ to R⁵ are either both hydrogen or one is hydrogen and the other alkyl. In compounds with one non-phenyl moiety like NH-pyrroles, furans and thiophens of formula I this non-phenyl moiety is hydrogen or alkyl.

According to the invention the compounds of formula I are 2,3,5-triphenyl pyrroles, -furans or -thiophenes, 1,2,4-triphenyl pyrroles, especially 1,2,4-triphenyl-5-alkyl pyrroles, 1,3,4-triphenyl pyrroles, 2,4,5-triphenyl pyrroles, -furans or -thiophenes or 3,4,5-triphenyl pyrroles, -furans or -thiophenes. Preferred compounds of the invention are 2,3,5-triphenyl pyrroles, -furans or -thiophenes, 1,2,4-triphenyl pyrroles and 1,3,4-triphenyl pyrroles.

According to the present invention the numbering of the atoms in the 5-membered heterocyclus is as follows: the heterocyclic atom is number one and aromatic substituents have higher priority than aliphatic substituents. Additionaly the numbering of aromatic substituents is as low as possible.

For a better understanding of the invention, in the following the chemical terms used are explained.

The term "alkyl" means C₁-C₆-alkyl, especially C₁-C₄-alkyl, preferably methyl, ethyl, n-propyl, i-propyl, n-butyl, i-butyl, t-butyl. "Alkoxy" denotes an alkyl group as described above bonded through an oxygen linkage (-O-). The term "alkoxy" preferably means C₁-C₄-alkoxy, more preferred methoxy or ethoxy. Halogen means F, Cl, Br, I, preferably chlor or flour. "Trihalomethyl" means preferably trichloro- or trifluoromethyl.

According to the invention the hydroxy groups in the substituents R¹-R⁵ can also be protected by protecting groups well known for hydroxy like for instance benzoyl, acetyl, benzyl or p-methoxybenzyl.

The above mentioned compounds of the invention include the pharmaceutically acceptable salts or esters, or any other compound which, upon administration to a human subject, is capable of providing (directly or indirectly) the therapeutically active metabolite.

Salts according to the invention which may be conveniently used in therapy include physiologically acceptable base salts, eg derived from an appropriate base, such as alkali metal (e.g. sodium) salts, alkaline earth metal (e.g. magnesium) salts or ammonium salts.

According to the invention the following 2,3,5-triphenyl compounds of formula I are especially preferred:
1) 2-(2-Chloro-4-methoxyphenyl)-3,5-bis(4-methoxyphenyl)-1*H*-pyrrole
2) 5-(2-Chloro-4-methoxyphenyl)-2,3-bis(4-methoxyphenyl)-1*H*-pyrrole
3) 2,5-Bis(2-Chloro-4-methoxyphenyl)-3-(4-methoxyphenyl)-1*H*-pyrrole
4) 2-(4-Chloro-2-methoxyphenyl)-3,5-bis(4-methoxyphenyl)-1*H*-pyrrole
5) 5-(2-Chloro-4-methoxyphenyl)-2-(4-chloro-2-methoxyphenyl)-3-(4-methoxyphenyl)-1*H*-pyrrole
6) 5-(4-Chloro-2-methoxyphenyl)-2,3-bis(4-methoxyphenyl)-4-propyl-1*H-*pyrrole
7) 2-(2-Chloro-4-hydroxyphenyl)-3,5-bis(4-hydroxyphenyl)-1*H*-pyrrole
8) 5-(2-Chloro-4-hydroxyphenyl)-2,3-bis(4-hydroxyphenyl)-1*H*-pyrrole
9) 2,5-Bis(2-Chloro-4-hydroxyphenyl)-3-(4-hydroxyphenyl)-1*H*-pyrrole
10) 2-(4-Chloro-2-hydroxyphenyl)-3,5-bis(4-hydroxyphenyl)-1*H*-pyrrole
11) 5-(2-Chloro-4-hydroxyphenyl)-2-(4-chloro-2-hydroxyphenyl)-3-(4-hydroxyphenyl)-1*H*-pyrrole
12) 5-(4-Chloro-2-hydroxyphenyl)-2,3-bis(4-hydroxyphenyl)-4-propyl-1*H-*pyrrole
13) 2,5-Bis(2-chloro-4-ethoxyphenyl)-3-(4-ethoxyphenyl)-1*H*-pyrrole
14) 2-(2-Fluoro-4-methoxyphenyl)-3,5-bis(4-methoxyphenyl)-1*H*-pyrrole
15) 2,3,5-Tris(2-chloro-4-methoxyphenyl)-1*H*-pyrrole
16) 2-(2-Chloro-4-methoxyphenyl)-5-(2-fluoro-4-methoxyphenyl)-3-(4-methoxyphenyl)-1*H*-pyrrole
17) 5-(2-Chloro-4-methylphenyl)-2,3-di-p-toluyl-1*H*-pyrrole
18) 3,5-Bis(4-methoxyphenyl)-2-(2-trifluoromethyl-4-methoxyphenyl)--1*H-*pyrrole
19) 2,5-Bis(2-chloro-4-methoxyphenyl)-3-(4-methoxyphenyl)-furan

The following 1,2,4-triphenyl compounds of formula I are also especially preferred compounds:
20) 4-(2-Chloro-4-methoxyphenyl)-1,2-bis(4-methoxyphenyl)-5-methyl-1*H-*pyrrole
21) 4-(2-Chloro-4-methoxyphenyl)-5-ethyl-1,2-bis(4-methoxyphenyl)-1*H-*pyrrole
22) 4-(2-Chloro-4-methoxyphenyl)-1,2-bis(4-methoxyphenyl)-5-propyl-1*H-*pyrrole
23) 4-(2-Chloro-4-methoxyphenyl)-5-isobutyl-1,2-bis(4-methoxyphenyl)-1*H-*pyrrole
24) 4-(2-Fluoro-4-methoxyphenyl)-1,2-bis(4-methoxyphenyl)-5-propyl-1*H-*pyrrole
25) 4-(2-Chloro-4-hydroxyphenyl)-1,2-bis(4-hydroxyphenyl)-5-methyl-1*H-*pyrrole
26) 4-(2-Chloro-4-hydroxyphenyl)-5-ethyl-1,2-bis(4-hydroxyphenyl)-1*H-*pyrrole
27) 4-(2-Chloro-4-hydroxyphenyl)-1,2-bis(4-hydroxyphenyl)-5-propyl-1*H-*pyrrole
28) 4-(2-Chloro-4-hydroxyphenyl)-5-isobutyl-1,2-bis(4-hydroxyphenyl)-1*H-*pyrrole
29) 4-(2-Fluoro-4-hydroxyphenyl)-1,2-bis(4-hydroxyphenyl)-5-propyl-1*H-*pyrrole
30) 4-(2-Chloro-4-propoxyphenyl)-1,2-bis(4-propoxyphenyl)-5-methyl-1*H-*pyrrole
31) 4-(2-Fluoro-4-methoxyphenyl)-1,2-bis(4-methoxyphenyl)-5-methyl-1*H-*pyrrole
32) 4-(2-Chloro-4-methoxyphenyl)-1,2-bis(4-methoxyphenyl)-1*H*-pyrrole
33) 1,2,4-Tris(2-chloro-4-methoxyphenyl)-1*H*-pyrrole
34) 1,2,4-Tris(2-chloro-4-methylphenyl)-1*H*-pyrrole
35) 1,2,4-Tris(2-chlorophenyl)-5-methyl-1*H*-pyrrole
36) 4-(3-Chloro-5-methoxyphenyl)-1,2-bis(4-methoxyphenyl)-5-methyl-1*H-*pyrrole
37) 4-(2-Chloro-4-methoxyphenyl)1,2-bis(4-methoxyphenyl)-3-methyl-1*H-*pyrrole

From the group of the 1,3,4-triphenyl pyrroles of formula I 1-(2-Chloro-4-methoxyphenyl)-3,4-bis(4-ethoxyphenyl)-1*H*-pyrrole is an especially preferred compound.

According to the invention one or more triphenyl modified 5-membered heterocycles of the general formula I are useful as anticancer or antiinflammatory agents due to their COX-inhibiting and antiproliferative effects. Therefore, the use of one or more compounds of formula I 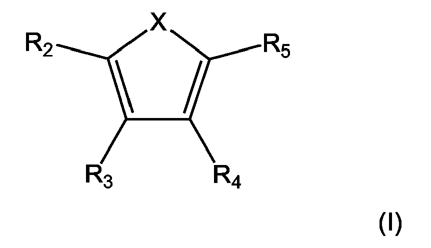 wherein
- X: represents NR¹, O or S
- R¹ to R⁵: independently from each other represent H, alkyl, unsubstituted phenyl or phenyl substituted with hydroxy, alkoxy, alkyl, trihalomethyl and/or halogen,
wherein at least one of the three phenyl moieties is substituted with halogen or trihalomethyl
or their salts or esters
for the manufacture of a pharmaceutical composition for prevention and treatment of cancer or inflammatory diseases is a further object of the present invention.

In a preferred aspect of the invention the compounds of formula I are suitable in the prevention and therapy of breast, colorectal, lungs, gastric, pancreatic and esophagal cancer and their metastases. A further proliferative disease which can be treated or prevented by the compounds of formula I is a neoplasia, such as for instance leukaemia. Leukemias include, but are not limited to, acute myelogenous leukemia, chronic myelogenous leukemia and juvenile myelomonocytic leukemia.

Inflammatory diseases which can be treated or prevented by the compounds of formula I include, but are not limited to, arthritis, gout, Crohn's disease, ulcerative colitis, rheumatism, especially rheumatoid arthritis and chronic hepatitis C.

Pharmaceutical compositions comprising in an amount sufficient to exhibit a therapeutic or preventive effect as active substance one or more triphenyl modified 5-membered heterocycles of general formula I 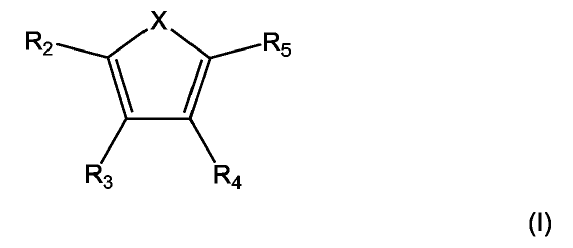 wherein
- X: represents NR¹, O or S
- R¹ to R⁵: independently from each other represent H, alkyl, unsubstituted phenyl or phenyl substituted with hydroxy, alkoxy, alkyl, trihalomethyl and/or halogen,
wherein at least one of the three phenyl moieties is substituted with halogen or trihalomethyl
or their salts or esters
are also an object of the present invention. The pharmaceutical compositions may also comprise conventional auxiliary substances, preferably carriers, adjuvants and/or vehicles. For example, said carriers can be fillers, extenders, binders, humectants, disintegrants, dissolution retarders, absorption enhancers, wetting agents, adsorbents, and/or lubricants.

The pharmaceutical compositions of the invention may be prepared as a gel, powder, tablet, sustained-release tablet, premix, emulsion, infusion formulation, drops, concentrate, granulate, syrup, pellet, bolus, capsule, aerosol, spray or inhalant and/or used in this form.

For example, the pharmaceutical composition of the present invention can be administered orally in any orally tolerable dosage form, including capsules, tablets and aqueous suspensions and solutions, without being restricted thereto. In case of tablets for oral application, carriers frequently used include microcrystalline cellulose, lactose and corn starch. Typically, lubricants such as magnesium stearate can be added. For oral administration in the form of capsules, useful diluents such as lactose and dried corn starch are employed. In oral administration of aqueous suspensions the active substance is combined with emulsifiers and suspending agents. Also, particular sweeteners and/or flavors and/or coloring agents can be added, if desired.

The compounds of formula I can also be present in micro-encapsulated form, optionally with one or more of the above-specified carriers.

In addition to the compounds of formula I as active substance(s), suppositories may include conventional water-soluble or water-insoluble carriers such as polyethylene glycols, fats, e.g. cocoa fat and higher esters (for example, C₁₄ alcohols with C₁₆ fatty acids) or mixtures of these substances.

In addition to the compounds of formula I as active substance(s), ointments, pastes, creams and gels may include conventional carriers such as animal and vegetable fats, waxes, paraffins, starch, tragacanth, cellulose derivatives, polyethylene glycols, silicones, bentonites, silicic acid, talc and zinc oxide or mixtures of these substances.

If the pharmaceutical composition according to the invention is provided as solution or emulsion it may include conventional carriers such as solvents, solubilizers, and emulsifiers such as water, ethyl alcohol, isopropyl alcohol, ethyl carbonate, ethyl acetate, benzyl alcohol, benzyl benzoate, propylene glycol, 1,3-butylene glycol, dimethylformamide, oils, especially cotton seed oil, peanut oil, corn oil, olive oil, castor oil and sesame oil, glycerol, glycerol formal, tetrahydrofurfuryl alcohol, polyethylene glycols, and fatty esters of sorbitan, or mixtures of these substances. For parenteral application, the solutions and emulsions may also be present in a sterile and blood-isotonic form.

In addition to the compounds of formula I as active substance(s), suspensions may include conventional carriers such as liquid diluents, e.g. water, ethyl alcohol, propylene glycol, suspending agents, e.g. ethoxylated isostearyl alcohols, polyoxyethylene-sorbitol and sorbitan esters, microcrystalline cellulose, aluminum metahydroxide, bentonite, agar, and tragacanth, or mixtures of these substances.

The pharmaceutical compositions can be present in the form of a lyophilized sterile injectable formulation, e.g. as a sterile injectable aqueous solution or aqueous or oily suspension. Such a suspension can also be formulated by means of methods known in the art, using suitable dispersing or wetting agents (such as Tween 80) and suspending agents.

The production of the pharmaceutical formulations specified above proceeds in a usual manner according to well-known methods, e.g. by mixing the active substance(s) with the carrier(s).

According to the invention the compounds of formula I are incorporated in a pharmaceutical formulation at a concentration of 0.1 to 99.5, preferably 0.5 to 95, and more preferably 20 to 80 wt.-%. That is, the active substance is present in the above pharmaceutical formulations, e.g. tablets, pills, granulates and others, at a concentration of preferably 0.1 to 99.5 wt.-% of the overall mixture.

According to an further embodiment, the present invention provides a method for treating cancer or an inflammatory disease comprising administering to a patient suffering from that disorder a therapeutically effective and safe amount of at least one compound of formula I. "Therapeutically effective amount" means an amount effective to yield the desired therapeutic response. For example, an amount effective to delay the growth of a cancer, to shrink or not metastasize. "Safe amount" refers to the quantity of a component that does not cause undue adverse side effects (such as toxicity, irritation, or allergic response) commensurate with a reasonable benefit/risk ratio when used in the manner of this invention.

Beside the prevention and treatment of COX-mediated diseases the compounds of formula I of the invention can be used in cell culture to interfere with COX-dependent functions such as prostaglandin E₂ (PGE₂) synthesis. Hence, the invention is also directed to the use of the triphenyl modified 5-membered heterocycles of formula I as COX-1 and/or COX-2 inhibitors, preferably as COX-2 inhibitors, for the investigation of cellular processes in biological in vitro, in vivo or ex vivo systems, e. g. cell cultures.

Methods to synthesize the compounds of formula I (pyrroles, furans and thiophens) are well-known in the art. The compounds of formula I can generally be prepared by cyclization of appropriately substituted 1,4-diketones. Ring substitution is for the most part determined by selection of the 1,4-diketone. The acid catalysed cyclization of 1,4-diketones to furans is for instance described in Wu, A. et al. (1997) Synthetic Comm. 27, 2087-2091). Treatment of appropriately substituted 1,4-diketones with Lawesson's Reagent give thiophenes (Kiebooms, R.H.L. et al. (1997) J. Org. Chem. 62, 1473-1480). Acid catalysed cyclazation of 1,4-diketones in the presence of a selected, appropriately substituted primary amine (for instance anilin, anisidine) results in the formation of N-substituted pyrroles (Khanna, I.K. et al. (1997) J. Med. Chem. 40, 1619-1633). Reaction of the 1,4-diketone with ammonium acetate in acetic acid results in a N-H pyrrole.

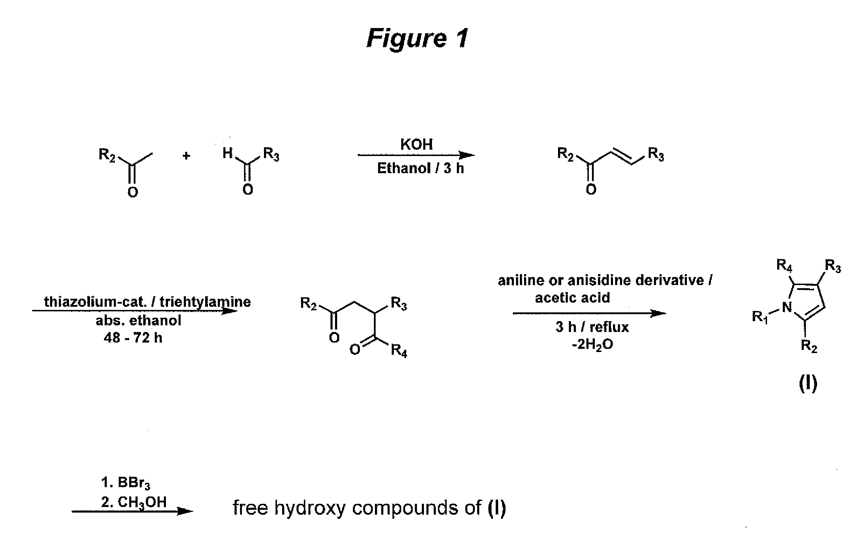

For the synthesis of the 1,4-diketones aldehydes and ketones that are commercially available or readily synthesized by well-known methods are treated with ethanolic KOH to form substituted α, β-unsaturated ketones. These are transformed using, for example, the Stetter reaction with appropriately substituted aldehydes in the presence of a thiazolium salt catalyst to the desired 1,4-diketones ((Khanna, I.K. et al. (1997) J. Med. Chem. 40, 1619-1633 and Stetter, H. (1976) Angewandte Chemie Int' I Ed. Eng. 15, 639-647)). Fig. 1 illustrates the synthesis of 1,2,4-triphenyl-1*H*-pyrroles of the invention using this approach. These compounds are especially preferred compounds.

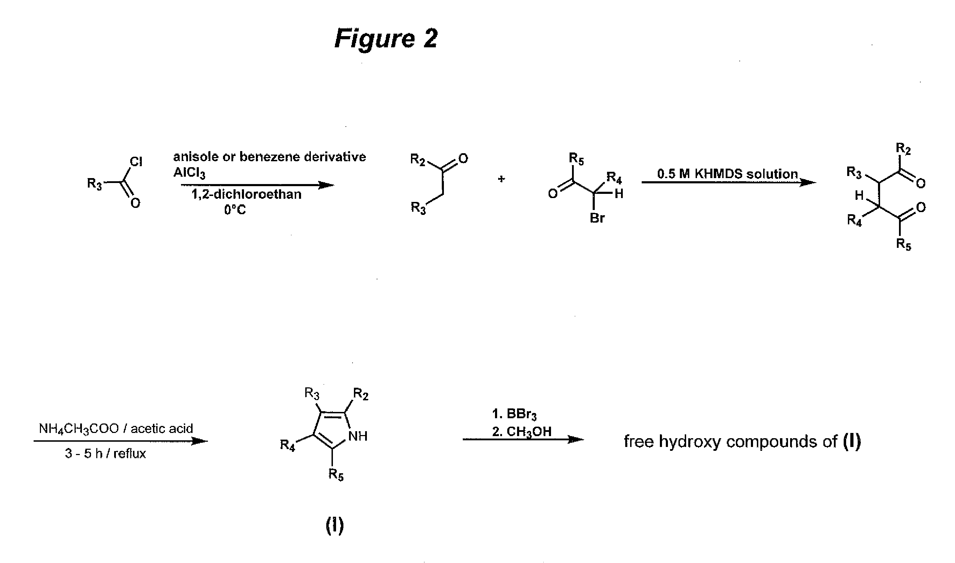

An alternate approach to 1,4-diketones uses enolate chemistry employing α-bromoketones as electrophiles. Fig. 2 illustrates the synthesis of 2,3,5-triphenyl-1*H*-pyrrols which are also especially preferred compounds of the invention using this alternate approach. Furans, thiophenes and pyrrols of formula I having substituents in one or more of the phenyl substituents can be deprotected with boron tribromide to afford the demethylated compounds.

According in the invention the compounds of formula I were investigated with respect to their COX-inhibiting, cytotoxic and estrogenic effects as described in the experimental part. As found by the inventors compounds of formula I with two 2-halogen-4-alkoxyphenyl groups and α 4-alkoxyphenyl group or three 2-halogen-4-alkoxyphenyl groups are the most COX-inhibitors. If one of the alkoxy groups is exchanged by hydroxy, estrogenic properties are detected. They increase with the number of hydroxy groups in the molecule, especially in 4-position of the phenyl moieties. Estrogenic properties are also detected, if one of the non-phenyl moieties R² to R⁵ in the molecule is alkyl.

Without intending to be limiting, the invention will be explained in more detail with reference to the following examples.

### General experimental procedure for synthesis of substituted 2,3,5-triphenyl-1-H-pyrroles (Examples 1 to 12)

### Synthesis of substituted diarylethanones (1)

1 eq. phenylacetic acid derivative was activated by reflux after the dropwise addition of 1 eq. thionylchloride under dry conditions for 45 min. After this, the reaction mixture was cooled to 4°C and was suspended in dry 1,2-dichloro-ethan (30 mL) without purification. After the suspension of 1.5 eq. AlCl₃, the benzene derivative (1 eq.) was added dropwise. The reaction mixture was allowed to warm to room temperature and was stirred for 1 h. The resultant product was hydrolyzed with ice (100mL) and neutralized with HCl and extracted with CH₂Cl₂ (3 x 50 mL). Solvent was removed under reduced pressure. Crude product was purified by flash column chromatography (diethyl ether/ligroine systems) to afford diarylethanones.

### Synthesis of substituted α-bromo ketones (2)

Aliphatic α-bromo acid was activated as described in (1) and reacted with 3-chloro anisole as shown in (1) and purified with flash column chromatography (diethyl ether/ligroine systems).

### Synthesis of substituted butane-1,4-diones (3)

A 0.5 M solution of KHMDS (1.1 eq.) was added to a stirring solution of diarylethanones (1 eq.). in THF(10 mL) at -78 °C. The mixture was stirred for 1 h followed by dropwise addition of α-bromo ketone (1.1 eq.). The reaction mixture was allowed to gradually warm to room temperature with stirring overnight and then quenched with the addition of H₂O (30 mL). The aqueous layer was washed with EtOAc (3 x 50 mL). The organic extracts were pooled and washed with water. The solvent was removed under reduced pressure. The residue was purified by flash column chromatography (diethyl ether/ligroine systems) to afford diones as a mixture of diastereomeres.

### Synthesis of methyl-protected 2,3,5-triphenyl-1H-pyrroles (4)

Substituted butane-1,4-dione (3) was soluted in dry acetic acid (20 mL). After addition of 9 eq. ammonium acetate the stirring mixture was refluxed for 3-5 h. After quenching with ice (50 ml) and neutralisation with NaHCO₃ (satured solution) the aqueous layer was extracted with CH₂Cl₂ (3 x 50mL). Pooled organic layer was neutralized with HCl, washed with H₂O and dried with Na₂SO₄. After removing the solvent the crude product was purified by flash column chromatography (ligroine/ethyl acetate or diethyl ether/ligroine systems) to afford 2,3,5-triaryl-1*H-*pyrroles.

### Synthesis of hydroxylated 2,3,5-triphenyl-1H-pyrroles (5)

To a stirring ice cooled solution of the protected 2,3,5-Triaryl-1*H*-pyrroles (4) in dry CH₂Cl₂ (15 ml) BBr₃ (soluted in 5 mL of CH₂Cl₂) was added dropwise. The reaction mixture was allowed to warm to room temperature by stirring overnight. After quenching with ice and stirring for 30 min the mixture was neutralized with NaHCO₃ (solution of 10%). Then the layers were separated and the aqueous layer was extracted with CH₂Cl₂ (3 x 50mL). After removing the solvent the crude product was purified by flash column chromatography (ligroine/ethyl acetate systems) to afford the demethylated products.

### Example 1

### Synthesis of 2-(2-Chloro-4-methoxyphenyl)-3,5-bis(4-methoxyphenyl)-1H-pyrrole

### a) 1-(2-Chloro-4-methoxyphenyl)-2-(4-methoxyphenyl)ethan-1-one

4-methoxyphenyl acetic acid (5.00 g, 30.09 mmol) was activated with SOCl₂ (2.18 mL, 30.09 mmol) and reacted with AlCl₃ (6.09 g, 45.13 mmol) and 3-chloroanisol (3.67 mL, 30.09 mmol) according to the general experimental procedure described above to afford 1-(2-Chloro-4-methoxyphenyl)-2-(4-methoxyphenyl)ethan-1-one.
C₁₆H₁₅ClO₃ (290.75)
light oil
**Yield:** 8.36 mmol (2.43 g), 28%
**Column chromatography**: **silicea** gel; ligroine/diethyl ether 2:1
¹H-NMR [(D₆)DMSO]: δ = 7.79 (d, 1H, J = 8.6, Ar*H*), 7.12 (d, 2H, J = 8.5, Ar*H*), 7.06 (d, 1H, J = 2.3, Ar*H*), 6.98 (dd, 1H, J = 2.5, J = 8.8, Ar*H*), 6.85 (d, 2H, J = 8.6, ArH), 4.19 (s, 2H, C*H*₂), 3.83 (s, 3H, OC*H*₃), 3.72 (s, 3H, OC*H*₃)
**MS** (El, 150°C): m/z (%) = 290 [M]^{+.} (4), 169 (100), 135 (50), 126 (6), 121 (13), 111 (3)
**IR** (film on KBR, cm-¹): 3058 (m), 2998 (m), 2937 (m), 2906 (m), 2834 (m), 2053 (m), 2024 (m),1691 (s), 1609 (m), 1593 (m),1514 (s), 1449 (m), 1335 (m), 1245 (s), 1179 (s), 1035 (s), 998 (m), 823 (m), 793 (s), 754 (s), 691 (s)

### b) 1-(2-Chloro-4-methoxyphenyl)-2,4-bis(4-methoxyphenyl)butane-1,4-dione

The reaction product of step a) (1.20 g, 4.13 mmol) was reacted with 0.5 M KHMDS (9.08 mL, 4.54 mmol) and 2-bromo-1-(4-methoxyphenyl)ethan-1-one (1.04 g, 4.54 mmol) at -78°C to afford the butane-1,4-dione.
C₂₅H₂₃ClO₅ (438.90)
brown oil
**Yield:** 2.37 mmol (1.04 g), 57%
**Column chromatography:** silicea gel; ligroine/diethyl ether 2:1
**MS** (EI, 220°C): m/z (%) = 438 [M]^{+**.**} (11), 420 (21), 169 (47), 135 (100), 111 (2), 77 (11)

### c) 2-(2-Chloro-4-methoxyphenyl)-3,5-bis(4-methoxyphenyl)-1H-pyrrole

Cyclisation of the reaction product of step b) (1.00 g, 2.28 mmol) using NH₄CH₃COO (1.58 g, 20.51 mmol) afforded the 2-(2-Chloro-4-methoxyphenyl)-3,5-bis(4-methoxyphenyl)-1*H*-pyrrole as a yellow solid.
C₂₅H₂₂ClNO₃ (419.90)
yellow solid, mp.: 162°C
**Yield:** 636 µmol (267 mg), 28%
**Column chromatography:** silicea gel; ligroine/diethyl ether 2:1
**¹H-NMR** [(D₆)DMSO]: δ = 11.04 (s, 1H, N*H*), 7.65 (d, 2H, J = 8.7, Ar*H*), 7.31 (d, 1H, J = 8.5, Ar*H*), 7.12 (d, 1H, J = 2.6, Ar*H*), 7.07 (d, 2H, J = 8.9, Ar*H*), 7.00 (dd, 1H, J = 2.6, J = 8.6, Ar*H*), 6.94 (d, 2H, J = 9.0, Ar*H*), ), 6.76 (d, 2H, J = 8.9, Ar*H*), 6.66 (d, 1H, J = 2.9, 4-H), 3.83 (s, 3H, OC *H*₃), 3.77 (s, 3H, OC*H*₃), 3.66 (s, 3H, OC *H*₃)
**MS** (EI, 150°C): m/z (%) = 419 [M]^{+**.**} (100), 405 (33), 391 (7), 326 (10), 210 [M]⁺⁺ (8), 135 (8), 77 (2)
**IR** (KBr, cm⁻¹): 3363 (m), 3027 (w), 2935 (w), 2836 (w), 1607 (m), 1591 (m), 1516 (s), 1497 (s), 1463 (m), 1286 (m), 1247 (s), 1179 (m), 1039 (m), 960 (w), 836 (m), 811 (w), 797 (w)

### Example 2

### Synthesis of 5-(2-Chloro-4-methoxyphenyl)-2,3-bis(4-methoxyphenyl)-1H-pyrrole

### a) 1,2-Bis(4-methoxyphenyl)ethan-1-one

4-methoxyphenyl acetic acid (10.00 g, 60.18 mmol) was activated with SOCl₂ (4.38 mL, 60.18 mmol) and reacted with AlCl₃ (12.18 g, 90.26 mmol) and anisole (6.55 mL, 60.18 mmol) according to the general experimental procedure described above to afford 1,2-Bis(4-methoxyphenyl)ethan-1-one.
C₁₆H₁₆0₃ (256.30)
white solid, mp.: 110°C
**Yield:** 41.55 mmol (10.65 g), 69%
**Column chromatography:** silicea gel; ligroine/diethyl ether 2:1
**¹H-NMR** [(D₆)DMSO]: δ = 8.01 (d, 2H, J = 8.8, Ar*H*), 7.17 (d, 2H, J = 8.5, Ar*H*), 7.02 (d, 2H, J = 8.8, Ar*H*), 6.88 (d, 2H, J = 8.5, Ar*H*), 4.22 (s, 2H, C*H*₂), 3.83 (s, 3H, OC*H*₃), 3.71 (s, 3H, OC*H*₃)
**MS** (El, 60°C): m/z (%) = 256 [M]^{+**·**} (13), 135 (100), 121 (8), 107 (4), 92 (3), 77 (8), 64 (2)
**IR** (KBr, cm⁻¹): 3035 (w), 2964 (m), 2238 (w), 2841 (m), 1676 (s), 1598 (s), 1599 (s), 1512 (s), 1466 (m), 1314 (m), 1266 (s), 1251 (s), 1223 (m), 1170 (s), 1115 (w), 1105 (w), 1026 (s), 992 (m), 831 (m), 809 (w), 780 (w)

### b) 2-Bromo-1-(2-chloro-4-methoxyphenyl)ethan-1-one

bromo acetic acid (7.00 g, 50.38 mmol) was activated with SOCl₂ (3.67 mL, 50.38 mmol) and reacted with AlCl₃ (10.06 g, 75.57 mmol) and 3-chloroanisole (6.15 mL, 50.38 mmol) according to the general experimental procedure described above to afford 2-Bromo-1-(2-chloro-4-methoxyphenyl)ethan-1-one.
C₉H₈BrClO₂ (263.52)
light oil
**Yield:** 26.87 mmol (7.08 g), 53%
**Column chromatography:** silicea gel; ligroine/diethyl ether 7:1
**¹H-NMR** [(D₆)DMSO]: δ = 7.88 (d, 1H, J = 8.5, Ar*H*), 7.15 (d, 1H, J = 2.3 Ar*H*), 7.04 (dd, 1H, J = 2.3, J = 8.6, Ar*H*), 4.82 (s, 2H, BrC*H*₂), 3.86 (s, 3H, OC*H*₃)
**MS** (EI, 60°C): m/z (%) = 262 [M]^{+**.**} (4), 169 (100), 155 (7), 126 (12), 77 (12), 63 (17)
**IR** (film on KBr, cm⁻¹): 3077 (w), 2926 (m), 2853 (w), 1696 (s), 1599 (s), 1461 (m) 1398 (w), 1314 (m),1297 (m), 1237 (s), 1193 (m),1054 (m), 1034 (m), 978 (w), 915 (w), 884 (m), 810 (m)

### c) 4-(2-Chloro-4-methoxyphenyl)-1,2-bis(4-methoxyphenyl)butane-1,4-dione

The reaction product of step a) (1.50 g, 5.85 mmol) was reacted with 0.5 M KHMDS (12.88 mL, 6.44 mmol) and the reaction product of step b) (1.70 g, 6.44 mmol) at -78°C to afford the butane-1,4-dione.
C₂₅H₂₃ClO₅ (438.90)
orange oil
**Yield:** 1.83 mmol (805 mg), 31%
**Column chromatography:** silicea gel; ligroine/diethyl ether 2:1
**MS** (El, 165°C): m/z (%) = 438 [M]^{+**·**} (12), 269 (4), 169 (36), 135 (100), 111 (1), 107 (4), 77 (11)

### d) 5-(2-Chloro-4-methoxyphenyl)-2,3-bis(4-methoxyphenyl)-1H-pyrrole

Cyclisation of the reaction product of step c) (800 mg, 1.82 mmol) using NH₄CH₃COO (1.26 g, 16.40 mmol) afforded the 5-(2-Chloro-4-methoxyphenyl)-2,3-bis(4-methoxyphenyl)-1*H*-pyrrole as a yellow solid.
C₂₅H₂₂ClNO₃ (419.90)
yellow solid, mp.: 66°C
**Yield:** 695 µmol (292 mg), 38%
**Column chromatography:** silicea gel; gradually eluation: ligroine/ethyl acetate 15:1 (1.5 l) ligroine/diethyl ether 2:1 (1.8 l)
**¹H-NMR** [(D₆)DMSO]: δ = 11.09 (s, 1H, N*H*), 7.61 (d, 1H, J = 8.7, Ar*H*), 7.31 (d, 2H, J = 8.7, Ar*H*), 7.24 (d, 2H, J = 8.6, Ar*H*), 7.10 (d, 1H, J = 2.5, Ar*H*), 6.99 (dd, 1H, J = 2.5, J = 8.7, Ar*H*), 6.91 (d, 2H, J = 8.7, Ar*H*), 6.84 (d, 2H, J = 8.7, Ar*H*),), 6.53 (d, 1H, J = 2.6, 4-H), 3.80 (s, 3H, OC*H*₃), 3.76 (s, 3H, OC*H*₃), 3.72 (s, 3H, OC*H*₃)
**MS** (EI, 330°C): m/z (%) = 419 [M]^{+**.**} (100), 405 (41), 391 (41), 210 [M]⁺⁺ (6), 135 (14), 77 (3)
**IR** (KBr, cm⁻¹): 3457 (m), 3002 (w), 2955 (w), 2956 (w), 2835 (w), 1609 (m), 1590 (w), 1511 (s), 1519 (m), 1494 (s), 1461 (m), 1288 (m), 1246 (s), 1177 (m), 1035 (m), 959 (w), 863 (w), 834 (m), 805 (w)

### Example 3

### Synthesis of 2,5-Bis(2-chloro-4-methoxyphenyl)-3-(4-methoxy-phenyl)-1H-pyrrole

### a) 1,2 Bis(2-chloro-4-methoxyphenyl)-2-(4-methoxyphenyl)butane-1,4-dione

1-(2-Chloro-4-methoxyphenyl)-2-(4-methoxyphenyl)ethan-1-one (Example 1, step a)) (1.30 g, 4.47 mmol) was reacted with KHMDS (9.84 mL, 4.92 mmol) and 2-Bromo-1-(2-chloro-4-methoxyphenyl)ethan-1-one (Example 2, step b)) (1.30 g, 4.92 mmol) at -78°C to afford the 1,2 Bis(2-chloro-4-methoxyphenyl)-2-(4-methoxyphenyl)butane-1,4-dione.
C₂₅H₂₂Cl₂O₅ (473.34)
gelbes Öl
**Yield:** 2.18 mmol (1.03 g), 49%
**Column chromatography:** silicea gel; ligroine/diethyl ether 2:1
**MS** (El, 165°C): m/z (%) = 472 [M]₊. (3), 454 (1), 303 (1), 169 (100), 135 (17), 111 (4), 77 (9)

### b) 2,5-Bis(2-chloro-4-methoxyphenyl)-3-(4-methoxyphenyl)-1H-pyrrole

Cyclisation of the reaction product of step a) (1.00 g, 2.11 mmol) using NH₄CH₃COO (1.47 g, 19.01 mmol) afforded the 2,5-Bis(2-chloro-4-methoxyphenyl)-3-(4-methoxyphenyl)-1*H*-pyrrole as a white solid.
C₂₅H₂₁Cl₂NO₃ (454.34)
white solid, mp.: 145°C
**Yield:** 295 µmol (134 mg), 14%
**Column chromatography:** silicea gel; ligroine/diethyl ether 2:1
**¹H-NMR** [(D₆)DMSO]: δ = 11.21 (s, 1H, N*H*), 7.57 (d, 1H, J = 8.7, Ar*H*), 7.31 (d, 1H, J = 8.5, Ar*H*), 7.14 (d, 1H, J = 2.5, Ar*H*), 7.11 (d, 1H, J = 2.5, Ar*H*), 7.05 (d, 2H, J = 8.6, Ar*H*), 6.98 (dd, 1H, J = 2.5, J = 8.6, Ar*H*), 6.96 (dd, 1H, J = 2.5, J = 8.5, Ar*H*),), 6.78 (d, 2H, J = 8.6, Ar*H*), 6.70 (d, 1H, J = 2.6, 4-H), 3.82 (s, 3H, OC*H*₃), 3.81 (s, 3H, OC*H*₃), 3.69 (s, 3H, OC*H*₃)
**MS** (EI, 200°C): m/z (%) = 453 [M]^{+**.**} (100), 438 (23), 227 [M]⁺⁺ (6), 169 (16), 135 (29)
**IR** (KBr, cm⁻¹): 3432 (m), 3028 (w), 2955 (w), 2958 (w), 2935 (w), 2836 (w), 1607 (m), 1586 (m), 1562 (w), 1515 (m), 1487 (s), 1462 (m), 1437 (m), 1285 (s), 1243 (s), 1218 (s), 1173 (m), 1105 (w), 1039 (s), 960 (w), 864 (w), 837 (m), 802 (m)

### Example 4

### Synthesis of 2-(4-Chloro-2-methoxyphenyl)-3,5-bis(4-methoxyphenyl)-1H-pyrrole

### a) 1-(4-Chloro-2-methoxphenyl)-2-(4-methoxyphenyl)ethan-1-one

4-methoxyphenyl acetic acid (5.00 g, 30.09 mmol) was activated with SOCl₂ (2.18 mL, 30.09 mmol) and reacted with AlCl₃ (6.09 g, 45.13 mmol) and 3-chloroanisole (3.67 mL, 30.09 mmol) according to the general experimental procedure described above to afford 1-(4-Chloro-2-methoxphenyl)-2-(4-methoxyphenyl)ethan-1-one.
C₁₆H₁₅ClO₃ (290.75)
brown solid, mp.: 57°C
**Yield:** 10.15 mmol (2.95 g), 34%
**Column chromatography:** silicea gel; ligroine/diethyl ether 2:1
¹H-NMR [(D₆)DMSO]: δ = 7.53 (d, 1H, J = 8.3, Ar*H*), 7.25 (d, 1H, J = 1.8, Ar*H*), 7.09 (dd, 1H, J = 1.8, J = 8.3, Ar*H*), 7.06 (d, 2H, J = 8.6, Ar*H*), 6.84 (d, 2H, J = 8.6, Ar*H*), 4.14 (s, 2H, C*H*₂), 3.92 (s, 3H, OC*H*₃), 3.70 (s, 3H, OC*H*₃)
**MS** (EI, 60°C): m/z (%) = 290 [M]^{+**.**} (3), 169 (40), 142 (100), 135 (14), 121 (17), 111 (52), 99 (27), 77 (6)
**IR** (KBr, cm⁻¹): 3108 (w), 3075 (w), 2952 (w), 2835 (w), 2359 (w), 1669 (s), 1588 (s),1515 (s), 1463 (m), 1401 (m),1330 (w), 1301 (m), 1244 (s), 1179 (m), 1099 (m), 1033 (m), 1017 (m), 934 (w), 879 (m), 846 (m), 826 (m)

### b) 1-(4-Chloro-2-methoxyphenyl)-2,4-bis(4-methoxyphenyl)butane-1,4-dione

The reaction product of step a) (1.20 g, 4.13 mmol) was reacted with KHMDS (9.08 mL, 4.54 mmol) and 2-Bromo-1-(4-methoxyphenyl)ethan-1-one (1.04 g, 4.54 mmol) at -78°C to afford the butane-1,4-dione.
C₂₅H₂₃ClO₅ (438.90)
brown oil
**Yield:** 2.71 mmol (1.19 g), 66%
**Column chromatography:** silicea gel; ligroine/diethyl ether 2:1
**MS** (EI, 250°C): m/z (%) = 438 [M]^{+**.**} (5), 169 (30), 135 (100), 107 (5), 77 (13)

### c) 2-(4-Chloro-2-methoxyphenyl)-3,5-bis(4-methoxyphenyl)-1H-pyrrole

Cyclisation of the reaction product of step b) (1.10 g, 2.51 mmol) using NH₄CH₃COO (1.74 g, 22.56 mmol) afforded the 2-(4-Chloro-2-methoxyphenyl)-3,5-bis(4-methoxyphenyl)-1*H*-pyrrole as a white solid.
C₂₅H₂₂ClNO₃ (419.90)
white solid, mp.: 125°C
**Yield:** 836 µmol (351 mg), 33%
**Column chromatography:** silicea gel; ligroine/diethyl ether 2:1
**¹H-NMR** [(D₆)DMSO]: δ = 11.01 (s, 1H, N*H*), 7.63 (d, 2H, J = 8.7, Ar*H*), 7.16 (d, 1H, J = 8.2, Ar*H*), 7.13 (d, 1H, J = 1.8, Ar*H*), 7.08 (d, 1H, J = 8.7, Ar*H*), 6.99 (dd, 1H, J = 1.9, J = 8.2, Ar*H*), 6.93 (d, 2H, J = 8.8, Ar*H*), ), 6.79 (d, 2H, J = 8.8, Ar*H*), 6.61 (d, 1H, J = 2.7, 4-H), 3.77 (s, 3H, OCH₃), 3.70 (s, 3H, OCH₃), 3.63 (s, 3H, OCH₃)
**MS** (EI, 180°C): m/z (%) = 419 [M]^{+**·**} (100), 405 (21), 391 (15), 210 [M]⁺⁺ (8), 135 (6), 77 (2)
**IR** (KBr, cm-¹): 3450 (m), 3000 (w), 2934 (w), 2834 (w), 1699 (w), 1612 (w), 1511 (s), 1492 (s), 1461 (m), 1441 (w), 1398 (w), 1248 (s), 1178 (m), 1030 (m), 956 (w), 867 (w), 834 (m), 798 (w)

### Example 5

### Synthesis of 5-(2-Chloro-4-methoxyphenyl)-2-(4-chloro-2-methoxyphenyl)-3-(4-methoxyphenyl)-1H-pyrrole

### a) 4-(2-Chloro-4-methoxyphenyl)-1-(4-chloro-2-methoxyphenyl)-2-(4-methoxyphenyl)butane-1,4-dione

1-(4-Chloro-2-methoxyphenyl)-2-(4-methoxyphenyl)ethan-1-one (Example 4, step a)) (1.20 g, 4.13 mmol) was reacted with KHMDS (9.08 mL, 4.54 mmol) and 2-Bromo-1-(2-chloro-4-methoxyphenyl)ethan-1-one (Example 2, step b)) (1.20 g, 4.54 mmol) at -78°C to afford the butane-1,4-dione.
C₂₅H₂₂Cl₂O₅ (473.34)
brown oil
**Yield:** 2.24 mmol (1.06 g), 54%
**Column chromatography:** silicea gel; ligroine/diethyl ether 2:1
**MS** (EI, 150°C): m/z (%) = 472 [M]^{+**.**} (2), 454 (1), 303 (1), 169 (100), 135 (14), 111 (3), 77 (11)

### b) 5-(2-Chloro-4-methoxyphenyl)-2-(4-chloro-2-methoxyphenyl)-3-(4-methoxyphenyl)-1H-pyrrole

Cyclisation of the reaction product of step a) (1.03 g, 2.18 mmol) using NH₄CH₃COO (1.51 g, 19.58 mmol) afforded the 5-(2-Chloro-4-methoxyphenyl)-2-(4-chloro-2-methoxyphenyl)-3-(4-methoxyphenyl)-1*H*-pyrrole as a white solid.
C₂₅H₂₁Cl₂NO₃ (454.34)
yellow solid, mp.: 49°C
**Yield:** 669 µmol (304 mg), 31%
**Column chromatography:** silicea gel; ligroine/diethyl ether 2:1
**¹H-NMR** [(D6)DMSO]: δ = 11.05 (s, 1H, N*H*), 7.55 (d, 1H, J = 8.9, Ar*H*), 7.17 (d, 1H, J = 7.9, Ar*H*), 7.14 (d, 1H, J = 1.6, Ar*H*), 7.11 (d, 1H, J = 2.7, Ar*H*), 7.09 (d, 2H, J = 8.7, Ar*H*), 6.99 (dd, 1H, J = 1.6, J = 7.9, Ar*H*), 6.98 (dd, 1H, J = 2.6, J = 8.9, Ar*H*), ), 6.80 (d, 2H, J = 8.6, Ar*H*), 6.62 (d, 1H, J = 2.7, 4-H), 3.81 (s, 3H, OC*H*₃), 3.70 (s, 3H, OC*H*₃), 3.63 (s, 3H, OC*H*₃)
**MS** (El, 120°C): m/z (%) = 453 [M]⁺' (16), 438 (4), 419 (6), 347 (14), 223 (100), 169 (46), 135 (38)
**IR** (KBr, cm⁻¹): 3445 (s), 3001 (w), 2955 (w), 2958 (w), 2935 (w), 2833 (w), 1609 (m), 1584 (m), 1564 (w), 1509 (m), 1483 (s), 1461 (m), 1439 (m), 1287 (s), 1242 (s), 1220 (s), 1175 (m), 1102 (w), 1030 (s), 957 (w), 866 (m), 836 (m), 807 (m)

### Example 6

### Synthesis of 2-(4-Chloro-2-methoxyphenyl)-4,5-bis(4-methoxyphenyl)-3-propyl-1H-pyrrole

### a) 2-Bromo-1-(4-chloro-2-methoxyphenyl)pentan-1-one

2-bromo valerianic acid (5.07 mL, 38.67 mmol) was activated with SOCl₂ (4.72 mL, 38.67 mmol) and reacted with AlCl₃ (7.72 g, 58.00 mmol) and 3-chloranisole (3.23 mL, 38.67 mmol) according to the general experimental procedure described above to afford 2-Bromo-1-(4-chloro-2-methoxyphenyl)pentan-1-one.
light oil
**Yield:** 15.73 mmol (4.80 g), 41%
**Column chromatography:** silicea gel; ligroine/diethyl ether 5:1
**¹H-NMR** [(D₆)DMSO]: δ = 7.63 (d, 1H, J = 8.4, ArH), 7.31 (d, 1H, J = 1.9, ArH), 7.14 (dd, 1H, J = 1.9, J = 8.4, ArH), 5.45 (pq, 1H, J = 8.1, C*H*BrCH₂CH₂CH₃), 3.93 (s, 3H, OC*H*₃), 2.14 - 1.96 (m, 1H, CHBrC*H*₂CH₂CH₃), 1.95 - 1.78 (m, 1H, CHBrC*H*₂CH₂CH₃), 1.57 - 1.28 (m, 2H, CHBrCH₂C*H*₂CH₃), 0.92 (t, 3H, J = 7.4, CHBrCH₂CH₂C*H*₃)
**MS** (El, 40°C): m/z (%) = 304 [M]^{+**·**} (1), 262 (1), 225 (1),169 (100), 142 (2), 111 (4), 75 (3)
**IR** (film on KBr, cm⁻¹): 2961 (s), 2871 (w), 2840 (w) 1679 (s), 1591 (w), 1462 (m), 1401 (m),1247 (s), 1097 (m), 1024 (m),971 (w), 878 (m), 840 (w)

### b) 1-(4-Chloro-2-methoxyphenyl)-3,4-bis-(4-methoxyphenyl)-2-propylbutan-1,4-dione

1,2-Bis(4-methoxyphenyl)ethan-1-on (Example 2, step a)) (1.50 g, 5.85 mmol) was reacted with KHMDS (12.88 mL, 6.44 mmol) and the reaction product of step a) (1.97 g, 6.44 mmol) at -78°C to afford the butane-1,4-dione.
C₂₈H₂₉ClO₅ (480.98)
yellow oil
**Yield:** 1.77 mmol (852 mg), 30%
**Column chromatography:** silicea gel; ligroine/diethyl ether 1:1
**MS** (EI, 100°C): m/z (%) = 256 (13), 169 (83), 135 (100), 121 (13), 92 (9), 77 (15)

### c) 2-(4-Chloro-2-methoxyphenyl)-4,5-bis(4-methoxyphenyl)-3-propyl-1H-pyrrole

Cyclisation of the reaction product of step b) (800 mg, 1.66 mmol) using NH₄CH₃COO (1.54 g, 14.97 mmol) afforded the 2-(4-Chloro-2-methoxyphenyl)-4,5-bis(4-methoxyphenyl)-3-propyl-1*H*-pyrrole as a white solid.
C₂₈H₂₈ClNO₃ (461.98)
yellow solid, mp.: 114°C
**Yield:** 353 µmol (163 mg), 21%
**Column chromatography:** silicea gel; ligroine/diethyl ether 2:1
**¹H-NMR** [(D₆)DMSO]: δ = 10.69 (s, 1H, N*H*), 7.31 (d, 1H, J = 8.1, Ar*H*), 7.13 (m, 5H, Ar*H*), 7.05 (dd, 1H, J = 2.0, J = 8.1, Ar*H*), 6.90 (d, 2H, J = 8.7, Ar*H*), 6.76 (d, 2H, J = 8.9, Ar*H*), 3.82 (s, 3H, *OCH₃),* 3.79 (s, 3H, OC*H*₃), 3.79 (s, 3H, *OCH₃),* 2.25 (t, 2H, J = 7.7, CH₂CH₂CH₃), 1.09 (m, 2H, CH₂C*H*₂CH₃), 0.58 (t, 3H, J = 7.3, CH₂CH₂C*H*₃)
**MS** (EI, 130°C): m/z (%) = 461 [M]^{+**.**} (100), 446 (7), 432 (26), 231 (14), 169 (20), 135 (25)
**IR** (KBr, cm⁻¹): 3452 (s), 2955 (w), 2930 (w), 2835 (w), 1601 (m), 1518 (s), 1490 (s), 1457 (m), 1245 (s), 1177 (m), 1102 (w), 1030 (m), 956 (w), 830 (m), 808 (w)

### Example 7

### Synthesis of 2-(2-Chloro-4-hydroxyphenyl)-3,5-bis(4-hydroxyphenyl)-1H-pyrrole

According to the general demethylation procedure described above 2-(2-Chloro-4-methoxyphenyl)-3,5-bis(4-methoxyphenyl)-1*H*-pyrrole (Example 1) (107 mg, 255 µmol) was demethylated with BBr₃ (126 µL, 1.33 mmol) to obtain 2-(2-Chloro-4-hydroxyphenyl)-3,5-bis(4-hydroxyphenyl)-1*H*-pyrrole.
C₂₂H₁₆ClNO₃ (377.08)
brown solid, mp.: 159°C
**Yield:** 247 µmol (93 mg), 97%
**Column chromatography:** silicea gel; ligroine/ethyl acetate 1:1
**¹H-NMR** [(D₆)DMSO]: δ = 11.00 (s, 1H, N*H*), 9.99 (s, 1H, OH), 9.33 (s, 1H, OH), 9.11 (s, 1H, OH), 7.50 (d, 2H, J = 8.7, Ar*H*), 7.15 (d, 1H, J = 8.3, Ar*H*), 6.94 (d, 2H, J = 8.6, Ar*H*), 6.89 (d, 1H, J = 2.4, Ar*H*), 6.76 (dd, 1H, J = 2.4, J = 8.2, Ar*H*), 6.72 (d, 2H, J = 8.7, Ar*H*), 6.57 (d, 2H, J = 8.6, Ar*H*), 6.51 (d, 1H, J = 2.8, 4-H)
**MS** (El, 190°C): m/z (%) = 377 [M]^{+**·**} (100), 342 (5), 341 (6), 311 (7), 189 [M]⁺⁺ (2), 171 (3) **IR** (KBr, cm⁻¹): 3415 (s), 2967 (w), 2933 (w), 1610 (m), 1578 (w), 1516 (s), 1498 (s), 1445 (w), 1374 (w), 1222 (s), 1172 (s), 1102 (w), 1035 (w), 834 (m)
**CHN** (%): Calc.: C 69.94 H 4.27 N 3.71 Found: C 69.23 H 4.08 N 4.19

### Example 8

### Synthesis of 5-(2-Chloro-4-hydroxyphenyl)-2,3-bis(4-hydroxyphenyl)-1H-pyrrole

According to the general demethylation procedure described above 5-(2-Chloro-4-methoxyphenyl)-2,3-bis(4-methoxyphenyl)-1*H*-pyrrole (Example 2) was demethylated with BBr₃ (69 µL, 725 µmol) to obtain 5-(2-Chloro-4-hydroxyphenyl)-2,3-bis(4-hydroxyphenyl)-1*H*-pyrrole.
C₂₂H₁₆ClNO₃ (377.08)
brown solid, mp.: 141°C
**Yield:** 111 µmol (42 mg), 81%
**Column chromatography:** silicea gel; ligroine/ethyl acetate 1:1
**¹H-NMR** [(D6)DMSO]: δ = 10.85 (s, 1H, N*H*), 9.87 (s, 1H, O*H*), 9.38 (s, 1H, O*H*), 9.17 (s, 1H, O*H*), 7.44 (d, 1H, J = 8.5, Ar*H*), 7.17 (d, 2H, J = 8.5, Ar*H*), 7.04 (d, 2H, J = 8.5, Ar*H*), 6.90 (d, 1H, J = 2.5, Ar*H*), 6.76 (dd, 1H, J = 2.5, J = 8.5, Ar*H*), 6.63 (d, 2H, J = 8.6, Ar*H*), 6.43 (d, 1H, J = 2.6, 4-H)
**MS** (EI, 195°C): m/z (%) = 377 [M]⁺' (100), 376 (5), 375 (5), 223 (2), 206 [M]⁺⁺ (4)
**IR** (KBr, cm-¹): 3424 (s), 2935 (w), 1612 (s), 1590 (w), 1513 (s), 1484 (s), 1440 (w), 1254 (m), 1223 (s), 1169 (s), 1089 (w), 890 (m), 805 (m)
**CHN** (%): Calc.: C 69.94 H 4.27 N 3.71, Found: C 70.44 H 3.79 N 4.11

### Example 9

### Synthesis of 2,5-Bis(2-chloro-4-hydroxyphenyl)-3-(4-hydroxyphenyl)-1H-pyrrole

According to the general demethylation procedure described above 2,5-Bis(2-chloro-4-methoxyphenyl)-3-(4-methoxyphenyl)-1*H*-pyrrole (Example 3) (80 mg, 176 mmol) was demethylated with BBr₃ (87 µL, 924 mmol) to obtain 2,5-Bis(2-chloro-4-hydroxyphenyl)-3-(4-hydroxyphenyl)-1*H*-pyrrole.
C₂₂H₁₅Cl₂NO₃ (412.26)
green solid, mp.: 207 °C
**Yield:** 136 µmol (56 mg), 77%
**Column chromatography:** silicea gel; ligroine/ethyl acetate 1:1
**¹H-NMR** [(D6)DMSO]: δ = 10.98 (s, 1H, N*H*), 9.99 (s, 1H, O*H*), 9.88 (s, 1H, O*H*), 9.12 (s, 1H, O*H*), 7.43 (d, 1H, J = 8.6, Ar*H*), 7.16 (d, 1H, J = 8.4, Ar*H*), 6.94 (d, 2H, J = 8.5, Ar*H*), 6.90 (d, 1H, J = 2.7, Ar*H*), 6.89 (d, 1H, J = 2.6, Ar*H*),6.77 (dd, 1H, J = 2.5, J = 8.6, Ar*H*), 6.74 (dd, 1H, J = 2.6, J = 8.5, Ar*H*), 6.57 (d, 2H, J = 8.3, Ar*H*), 6.45 (d, 1H, J = 2.6, 4-*H*)
**MS** (EI, 210°C): m/z (%) = 411 [M]^{+**.**} (100), 376 (7), 375 (7), 223 (5), 206 [M]⁺⁺ (6), 188 (6), 171 (8)
**IR** (KBr, cm⁻¹): 3436 (s), 2950 (w), 2938 (w), 1614 (m), 1585 (w), 1572 (w), 1508 (s), 1485 (s), 1439 (w), 1376 (w), 1255 (m), 1224 (s), 1171 (s), 1093 (w), 890 (m), 806 (m)
**CHN** (%): Calc.: C 64.09 H 3.67 N 3.40, Found: C 64.48 H 4.03 N 3.99

### Example 10

### Synthesis of 2-(4-Chloro-2-hydroxyphenyl)-3,5-bis(4-hydroxyphenyl)-1H-pyrrole

According to the general demethylation procedure described above (2-(4-Chloro-2-methoxyphenyl)-3,5-bis(4-methoxyphenyl)-1*H*-pyrrole (Example 4) (100 mg, 238 µmol) was demethylated with BBr₃ (118 µL, 1.25 mmol) to obtain 2-(4-Chloro-2-hydroxyphenyl)-3,5-bis(4-hydroxyphenyl)-1*H*-pyrrole.
C₂₂H₁₆CIN0₃ (377.08)
brown solid, mp.: 173 °C
**Yield:** 191 µmol (72 mg), 80%
**Column chromatography:** silicea gel; ligroine/ethyl acetate 1:1
**¹H-NMR** [(D₆)DMSO]: δ = 10.82 (s, 1H, N*H*), 9.92 (s, 1H, *OH),* 9.34 (s, 1H, O*H*), 9.12 (s, 1H, O*H*), 7.49 (d, 2H, J = 8.5, Ar*H*), 7.06 (d, 1H, J = 8.1, Ar*H*), 7.02 (d, 2H, J = 8.5, Ar*H*), 6.92 (d, 1H, J = 1.9, Ar*H*), 6.81 (dd, 1H, J = 2.2, J = 8.3, Ar*H*), 6.74 (d, 2H, J = 8.5, Ar*H*), 6.60 (d, 2H, J = 8.5, Ar*H*), 6.45 (d, 1H, J = 2.6, 4-*H*)
**MS** (EI, 220°C): m/z (%) = 377 [M]^{+**.**} (100), 342 (1), 341 (2), 223 (6), 189 [M]⁺⁺ (3), 171 (6)
**IR** (KBr, cm⁻¹): 3422 (s), 3028 (w), 2957 (w), 2925 (w), 1613 (m), 1587 (w), 1514 (s), 1495 (s), 1447 (w), 1412 (w), 1374 (w), 1259 (s), 1172 (s), 1094 (w), 1044 (w), 836 (m)
**CHN** (%): Calc.: C 69.94 H 4.27 N 3.71, Found: C 70.40 H 4.97 N 3.49

### Example 11

### Synthesis of 5-(2-Chloro-4-hydroxyphenyl)-2-(4-chloro-2-hydroxyphenyl)-3-(4-hydroxyphenyl)-1H-pyrrole

According to the general demethylation procedure described above 5-(2-Chloro-4-methoxyphenyl)-2-(4-chloro-2-methoxyphenyl)-3-(4-hydroxyphenyl)-1*H*-pyrrole (Example 5) (100 mg, 220 µmol) was demethylated with BBr₃ (94 µL, 990 µmol) to obtain 5-(2-Chloro-4-hydroxyphenyl)-2-(4-chloro-2-hydroxyphenyl)-3-(4-hydroxyphenyl)-1*H*-pyrrole.
C₂₂H₁₅Cl₂NO₃ (412.26)
brown solid, mp.: 106°C
**Yield:** 189 µmol (78 mg), 86%
**Column chromatography:** silicea gel; ligroine/ethyl acetate 1:1
**¹H-NMR** [(D₆)DMSO]: δ = 10.85 (s, 1H, N*H*), 10.09 (s, 1H, OH), 9.89 (s, 1H, OH), 9.15 (s, 1H, OH), 7.46 (d, 1H, J = 8.6, Ar*H*), 7.08 (d, 1H, J = 8.3, Ar*H*), 7.01 (d, 2H, J = 8.5, Ar*H*), 6.92 (d, 1H, J = 1.7, Ar*H*), 6.89 (d, 1H, J = 2.4, Ar*H*),6.78 (m, 2H, Ar*H*), 6.63 (d, 2H, J = 8.5, Ar*H*), 6.53 (d, 1H, J = 2.5, 4-H)
**MS** (EI, 180°C): m/z (%) = 411 [M]^{+.} (100), 376 (2), 375 (3), 223 (9), 206 [M]⁺⁺ (3), 188 (7), 171 (2)
**IR** (KBr, cm⁻¹): 3429 (s), 3026 (w), 2956 (w), 2924 (w), 2855 (w), 1609 (m), 1588 (w), 1571 (w), 1511 (s), 1488 (s), 1457 (w), 1439 (w), 1378 (w), 1260 (m), 1223 (s), 1173 (s), 1096 (w), 1037 (w), 892 (m), 810 (m)
**CHN** (%): Calc.: C 64.09 H 3.67 N 3.40, Found: C 65.30 H 3.71 N 3.87

### Example 12

### Synthesis of 2-(4-Chloro-2-hydroxyphenyl)-4,5-bis(4-hydroxyphenyl)-3-propyl-1H-pyrrole

According to the general demethylation procedure described above 2-(4-Chloro-2-methoxyphenyl)-4,5-bis(4-methoxyphenyl)-3-propyl-1*H*-pyrrole (Example 6) (50 mg, 108 µmol) was demethylated with BBr₃ (54 µL, 568 µmol) to obtain 2-(4-Chloro-2-hydroxyphenyl)-4,5-bis(4-hydroxyphenyl)-3-propyl-1*H*-pyrrole.
C₂₅H₂₇ClNO₃ (419.90)
brown solid, mp.: 124°C
**Yield:** 102 µmol (43 mg), 95%
**Column chromatography:** silicea gel; ligroine/ethyl acetate 1:1
**¹H-NMR** [(D6)DMSO]: δ = 10.50 (s, 1H, N*H*), 9.97 (s, 1H, OH), 9.23 (s, 2H, OH), 7.24 (d, 2H, J = 8.2, Ar*H*), 7.02 (d, 2H, J = 8.6, Ar*H*), 6.96 (d, 2H, J = 8.4, Ar*H*), 6.94 (d, 1H, J = 2.1, Ar*H*), 6.89 (dd, 1H, J = 2.1, J = 8.1, Ar*H*), 6.72 (d, 2H, J = 8.4, Ar*H*), 6.56 (d, 2H, J = 8.6, Ar*H*), 2.29 (t, 2H, J = 7.7, CH₂CH₃CH₃), 1.10 (m, 2H , CH₂CH₂CH₃), 0.50 (t, 3H, J = 7.3, CH₂CH₂CH₃)
**MS** (El, 220°C): m/z (%) = 419 [M]⁺' (100), 390 (40), 355 (11), 210 [M]⁺⁺ (6), 178 (4)
**IR** (KBr, cm⁻¹): 3410 (s), 3048 (w), 2960 (w), 2931 (w), 1609 (s), 1513 (s), 1497 (s), 1452 (w), 1440 (w), 1375 (w), 1343 (w), 1261 (s), 1228 (m), 1173 (m), 1096 (w), 836 (m)
**CHN** (%): Calc.: C 71.51 H 5.28 N 3.34, Found: C 71.01 H 4.80 N 4.22

### General experimental procedure for synthesis of substituted 1,2,4-triphenyl-1-H-pyrroles (Example 13 to 22)

### Synthesis of 1,3-diphenylpropenones (1)

To a stirring solution of phenylethanone derivative (1 eq.) in dry ethanol (100 mL) KOH (1.5 eq.) was added. After the reflux of 30 min the mixture was coold to room temperature and the benzaldehyde derivative (1.2 eq.) was added dropwise. The crystals that formed were filtered and recrystallized from EtOH to afford 1,3-diarylpropenones (1).

### Synthesis of 1,3-diphenyl-1,4-dione (2)

The 1,3-diphenyl-1,4-diones (2) were prepared by the well-known Stetter method. To a stirring solution of 1,3-diphenypropenone derivative (1) (1 eq.) in dry ethanol were added the aliphatic aldehyde (3 eq.), 3-benzyl-5-(2-hydroxyethyl)-4-methylthiazoliumchloride (0.3 eq.) and triethylamine (4 eq.). The reaction mixture was refluxed for 36-72 h (dependent on starting material). The reaction mixture was cooled, diluted with CHCl₃ and washed with 1 % HCl. Poduct isolation with CHCl₃ (3 x 50 mL) and purification by flash column chromatography (ligroine/CH₂Cl₂ systems) afforded the corresponding 1,4-diketone as light oil.

### Synthesis of N-phenylated 1,2,4-triphenyl-1H-pyrroles (3)

To a stirring solution of 1,3-diphenyl-1,4-dione (1 eq.) in dry acetic acid (20 mL) was added 4-methoxyphenylamin. The mixture was refluxed for 3 h, cooled to room temperature, quenched with ice and neutralized with al aqueous solution of NaHCO₃ (10%). The layers were separated and the aqueous layer was washed with CH₂Cl₂ (3 x 50 mL). Pooled organic layer was neutralized with HCl, washed with H₂O and dried with Na₂SO₄. The crude product was purified by recrystallisation with 2-Propanol to afford white, yellow or brown solid.

### Synthesis of hydroxylated 1,2,4-triphenyl-1H-pyrroles (4)

To a stirring ice cooled solution of the protected 1,2,4-triphenyl-1*H*-pyrroles (3) in dry CH₂Cl₂ (15 mL) BBr₃ (soluted in 5 mL of CH₂Cl₂) was added dropwise. The reaction mixture was allowed to warm to room temperature by stirring overnight. After quenching with ice and stirring for 30 min the mixture was neutralized with NaHCO₃ (solution of 10%). Then the layers were separated and the aqueous layer was extracted with CH₂Cl₂ (3 x 50mL). After removing the solvent the crude product was purified by flash column chromatography (ligroine/ethyl acetate systems) to afford the demethylated products.

### Example 13

### Synthesis of 4-(2-Chloro-4-methoxyphenyl)-1,2-bis(4-methoxyphenyl)-5-methyl-1H-pyrrole

### a) 3-(2-Chlor-4-methoxyphenyl)-1-(4-methoxyphenyl)propenon

1-(4-methoxyphenyl)ethanone (3.70 g, 24.64 mmol) was reacted with KOH (2.07 g, 36.96 mmol) and 2-chloro-4-methoxybenzaldehyde (4.62 g, 27.10 mmol) according to the general experimental procedure described above to afford 3-(2-Chlor-4-methoxyphenyl)-1-(4-methoxyphenyl)propenon.
C₁₇H₁₅ClO₃ (302.75)
yellow solid, mp.: 118°C
**Yield:** 14.90 mmol (4.51 g), 60%
**¹H-NMR** [(D₆)DMSO]: δ = 8.18 (m, 3H, Ar*H*), 7.99 (d, 1H, J = 15.5, CH), 7.90 (d, 1H, J = 15.5, CH), 7.17 (d, 1H, J = 2.5, Ar*H*), 7.10 (d, 2H, J = 8.8, Ar*H*), 7.03 (dd, 1H, J = 2.4, J = 8.8, Ar*H*), 3.90 (s, 3H, OCH₃), 3.85 (s, 3H, OCH₃)
**MS** (El, 100°C): m/z (%) = 302 [M]^{+**.**} (5), 267 (100), 252 (4), 238 (3), 224 (4), 135 (20), 77 (10)
**IR** (KBr, cm⁻¹): 3075 (w), 2972 (w), 2934 (w), 2840 (w), 1648 (m), 1604 (s), 1575 (m), 1494 (m), 1460 (w), 1438 (m), 1423 (m), 1401 (w), 1339 (w), 1288 (m), 1268 (s), 1234 (s), 1205 (w), 1191 (m), 1109 (w), 1029 (m), 981 (m), 867 (m), 746 (m), 822 (m), 746 (w), 670 (w), 636 (w), 614 (w)

### b) 3-(2-Chloro-4-methoxyphenyl)-1-(4-methoxyphenyl)pentane-1,4-dione

The reaction product of step a) (1.50 g, 4.95 mmol) was refluxed with acetaldehyde (839 µL, 14.86 mmol), 3-benzyl-5-(2-hydroxyethyl)-4-methylthiazoliumchloride (401 mg, 1.49 mmol) and Triethylamine (2.76 ml, 19.82 mmol) for 36 h to afford 3-(2-Chloro-4-methoxyphenyl)-1-(4-methoxyphenyl)pentane-1,4-dione.
C₁₉H₁₉ClO₄ (346.80)
yellow oil
**Yield:** 1.25 mmol (435 mg), 25%
**Column chromatography:** silicea gel; ligroine/CH2C12 3:4
**¹H-NMR** [CDCl₃]**:** δ = 7.92 (d, 2H, J = 8.9, Ar*H*), 7.13 (d, 1H, J = 8.7, Ar*H*), 6.99 (d, 1H, J = 2.6, Ar*H*), 6.91 (d, 2H, J = 8.8, Ar*H*), 6.79 (dd, 1H, J = 2.7, J = 8.7, Ar*H*), 4.87 (dd, 1H, J = 3.8, J = 9.8, CH₂CH), 3.91 (dd, 1H, J = 9.8, J = 17.8, CH₂CH), 3.86 (s, 3H, OC*H*₃), 3.79 (s, 3H, OC*H*₃), 3.05 (dd, 1H, J = 3.8, J = 17.8, CH₂CH), 2.20 (s, 3H, CH₃)
**MS** (El, 140°C): m/z (%) = 346 [M]^{+**·**} (15), 269 (29), 135 (100), 107 (6), 77 (12)
**IR** (film on KBr, cm⁻¹): 3004 (w), 2961 (w), 2938 (w), 2912 (w), 2839 (w), 1716 (s), 1673 (s), 1601 (s), 1575 (m), 1496 (s), 1462 (w), 1440 (w), 1420 (w), 1355 (m), 1307 (w), 1290 (m), 1257 (s), 1239 (w), 1170 (s), 1113 (w), 1039 (s), 995 (w), 838 (m)

### c) 4-(2-Chloro-4-methoxypheny)-1,2-bis(4-methoxypheny))-5-methy)-1H-pyrrole

The reaction product of step b) (430 mg, 1.24 mmol) was refluxed with 4-methoxyphenylamin (993 mg, 8.06 mmol) to afford 4-(2-Chloro-4-methoxyphenyl)-1,2-bis(4-methoxyphenyl)-5-methyl-1H-pyrrole.
C₂₆H₂₄ClNO₃ (433.93)
yellow solid, mp: 69°C
**Yield:** 445 µmol (193 mg), 36%
**¹H-NMR** [(D₆)DMSO]: δ = 7.31 (d, 1H, J = 8.6, Ar*H*), 7.18 (d, 2H, J = 8.8, Ar*H*), 7.10 (d, 1H, J = 2.5, Ar*H*), 7.00 (d, 2H, J = 8.7, Ar*H*), 6.99 (d, 2H, J = 8.9, Ar*H*), 6.96 (dd, 1H, J = 2.5, J = 8.6, Ar*H*), 6.76 (d, 2H, J = 8.8, Ar*H*), 6.29 (s, 1H, 4-H), 3.80 (s, 3H, *OCH₃),* 3.79 (s, 3H, *OCH₃),* 3.68 (s, 3H, *OCH₃),* 1.92 (s, 3H, *CH₃)*
**MS** (EI, 70°C): m/z (%) = 333 [M]^{+**.**} (100), 418 (19), 217 [M]⁺⁺ (10), 77 (6)
**IR** (KBr, cm⁻¹): 3000 (w), 2955 (w), 2936 (w), 2912 (w), 2836 (w), 1609 (m), 1565 (w), 1513 (s), 1495 (s), 1463 (m), 1400 (m), 1379 (w). 1286 (m), 1248 (s), 1208 (w), 1179 (m), 1108 (w), 1056 (w), 1036 (m), 835 (m), 796 (w), 756 (m)

### Example 14

### Synthesis of 4-(2-Chloro-4-methoxyphenyl)-5-ethyl-1,2-bis(4-methoxyphenyl)-1H-pyrrole

### a) 3-(2-Chloro-4-methoxyphenyl)-1-(4-methoxyphenyl)hexane-1,4-dione

1-(2-Chloro-4-methoxyphenyl)-3-(4-methoxyphenyl)propenone (Example 13, step a)) (1.50 g, 4.95 mmol) was refluxed with propionaldehyde (1.08 mL, 14.86 mmol), 3-benzyl-5-(2-hydroxyethyl)-4-methylthiazoliumchloride (401 mg, 1.49 mmol) und triethylamine (2.76 mL, 19.82 mmol) for 48 h to afford 3-(2-Chloro-4-methoxyphenyl)-1-(4-methoxyphenyl)hexane-1,4-dione.
C₂₀H₂₁ClO₄ (360.83)
brown oil
**Yield:** 1.81 mmol (654 mg), 37%
**Column chromatography:** silicea gel; ligroine/diethyl ether 1:1
**¹H-NMR** [CDCl₃]: δ = 7.96 (d, 2H, J = 8.8, Ar*H*), 7.17 (d, 1H, J = 8.7, Ar*H*), 7.09 (d, 1H, J = 2.7, Ar*H*), 7.01 (d, 2H, J = 8.9, Ar*H*), 6.93 (dd, 1H, J = 2.7, J = 8.7, Ar*H*), 4.72 (dd, 1H, J = 4.1, J = 9.7, *CH₂CH),* 3.87 (dd, 1H, J = 9.7, J = 17.9, CH₂C*H*), 3.84 (s, 3H, *OCH₃),* 3.77 (s, 3H, *OCH₃),* 3.13 (dd, 1H, J = 4.1, J = 17.9, C*H*₂CH), 2.60 - 2.56 (m, 2H, *CH₂CH₃),* 0.83 (t, 3H, J = 7.4, *CH₂CH₃)*
**MS** (EI, 110°C): m/z (%) = 360 [M]^{+**.**} (12), 325 (1), 303 (3), 304 (3), 269 (19), 135 (100), 77 (9)
**IR** (film on KBr, cm⁻¹): 3056 (w), 2969 (w), 2936 (w), 2839 (w), 1714 (m), 1673 (m), 1601 (s), 1575 (w), 1496 (m), 1461 (m), 1441 (w), 1420 (w), 1353 (w), 1306 (w), 1287 (w), 1256 (s), 1237 (m), 1169 (m), 1114 (m), 1032 (m), 992 (w), 838 (m), 807 (w)

### b) 4-(2-Chloro-4-methoxyphenyl)-5-ethyl-1,2-bis(4-methoxyphenyl)-1H-pyrrole

The reaction product of step a (480 mg, 1.33 mmol) was refluxed with 4-methoxyphenylamin (1.06 g, 8.65 mmol) to afford 4-(2-Chloro-4-methoxyphenyl)-5-ethyl-1,2-bis(4-methoxyphenyl)-1*H*-pyrrole.
C₂₇H₂₆ClNO₃ (447.95)
white solid, mp: 89°C
**Yield:** 489 µmol (219 mg), 37%
**¹H-NMR** [(D₆)DMSO]: δ = 7.31 (d, 1H, J = 7.3, Ar*H*), 7.20 (d, 2H, J = 8.8, Ar*H*), 7.10 (d, 1H, J = 2.5, Ar*H*), 7.01 (d, 4H, J = 8.8, Ar*H*), 6.94 (dd, 1H, J = 2.6, J = 8.6, Ar*H*), 6.94 (d, 2H, J = 8.7, Ar*H*), 6.74 (d, 2H, J = 8.7, Ar*H*), 6.24 (s, 1H, 4-H), 3.82 (s, 3H, OC*H*₃), 3.77 (s, 3H, OC*H*₃), 3.66 (s, 3H, OC*H*₃), 2.39 (q, 2H, J = 7.4, C*H*₂CH₃), 0.67 (t, 3H, J = 7.4, CH₂C*H*₃)
**MS** (El, 200°C): m/z (%) = 447 [M]^{+**·**} (100), 432 (49), 418 (15), 224 [M]⁺⁺ (9), 77 (7), 28 (21)
**IR** (KBr, cm⁻¹): 3069 (w), 3000 (w), 2961 (w), 2932 (w), 2836 (w), 1606 (m), 1568 (w), 1514 (s), 1495 (s), 1463 (m), 1440 (m). 1381 (w), 1286 (m), 1240 (s), 1229 (m), 1180 (m), 1038 (m), 870 (w), 838 (m)

### Example 15

### Synthesis of 4-(2-Chloro-4-methoxyphenyl)-1,2-bis(4-methoxyphenyl)-5-propyl-1H-pyrrole

### a) 3-(2-Chloro-4-methoxyphenyl)-1-(4-methoxyphenyl)heptane-1,4-dione

3-(2-Chloro-4-methoxyphenyl)-1-(4-methoxyphenyl)propenone (Example 13, step a)) (2.50 g, 8.26 mmol) was refluxed with butyraldehyde (2.23 mL, 24.78 mmol), 3-benzyl-5-(2-hydroxyethyl)-4-methylthiazoliumchloride (668 mg, 2.48 mmol) und triethylamine (4.60 mL, 33.03 mmol) for 72 h to afford 3-(2-Chloro-4-methoxyphenyl)-1-(4-methoxyphenyl)heptane-1,4-dione.
C₂₁H₂₃ClO₄ (374.86)
yellow oil
**Yield:** 5.66 mmol (2.12 g), 68%
**Column chromatography:** silicea gel; ligroine/diethyl ether 2:1
**¹H-NMR** [CDCl₃]: δ = 7.92 (d, 2H, J = 8.8, Ar*H*), 7.11 (d, , J = 8.6, Ar*H*), 6.98 (d, 1H, J = 2.7, Ar*H*), 6.88 (d, 2H, J = 8.9, Ar*H*), 6.79 (dd, 1H, J = 2.7, J = 8.7, Ar*H*), 4.86 (dd, 1H, J = 3.6, J = 10.0, CH₂CH), 3.91 (dd, 1H, J = 10.0, J = 17.9, CH₂CH), 3.86 (s, 3H, OCH₃), 3.79 (s, 3H, OCH₃), 3.04 (dd, 1H, J = 3.7, J = 17.7, *CH₂CH),* 2.61 (ddd, 1H, J = 11.0, J = 8.3, J = 2.2, CH₂CH₂CH₃), 2.42 (ddd, 1H, J = 11.0, J = 8.3, J = 2.2, CH₂CH₂CH₃), 1.72 - 1.41 (m, 2H, CH₂CH₂CH₃), 0.84 (t, 3H, J = 7.3, CH₂CH₂CH₃)
**MS** (EI, 100°C): m/z (%) = 374 [M]^{+**.**} (10), 304 (3), 269 (29), 135 (100), 71 (13), 43 (21)
**IR** (film on KBr, cm⁻¹): 3069 (w), 2963 (m), 2936 (m), 2875 (w), 1713 (s), 1675 (s), 1602 (s), 1575 (m), 1511 (m), 1497 (s), 1462 (m), 1441 (w), 1422 (w), 1401 (w), 1361 (m), 1306 (w), 1258 (s), 1238 (m), 1171 (s), 1124 (w), 1077 (w), 1036 (m), 986 (w), 841 (w)

### b) 4-(2-Chloro-4-methoxyphenyl)-1,2-bis(4-methoxyphenyl)-5-propyl-1H-pyrrole

The reaction product of step a) (400 mg, 1.07 mmol) was refluxed with 4-methoxyphenylamin (854 mg, 6.94 mmol) to afford 4-(2-Chloro-4-methoxyphenyl)-1,2-bis(4-methoxyphenyl)-5-propyl-1*H*-pyrrole.
C₂₈H₂₈CIN0₃ (461.98)
yellow solid, mp: 121 °C
**Yield:** 387 µmol (179 mg), 36%
**¹H-NMR** [(D₆)DMSO]: δ = 7.31 (d, 1H, J = 8.5, Ar*H*), 7.17 (d, 2H, J = 8.7, Ar*H*), 7.10 (d, 1H, J = 2.4, Ar*H*), 6.99 (d, 4H, J = 8.8, Ar*H*), 6.94 (d, 1H, J = 2.5, Ar*H*), 6.74 (d, 2H, J = 8.7, Ar*H*), 6.24 (s, 1H, 4-H), 3.79 (s, 3H, OCH₃), 3.67 (s, 3H, OCH₃), 2.37 (t, 2H, J = 7.5, CH₂CH₂CH₃), 1.05 (m, 2H, CH₂CH₂CH₃) 0.51 (t, 3H, J = 7.3, CH₂CH₂CH₃)
**MS** (El, 150°C): m/z (%) = 461 [M]^{+**.**} (89), 432 (100), 397 (13), 231 [M]⁺⁺ (6), 57 (14), 28 (22)
**IR** (KBr, cm⁻¹): 3068 (w), 2960 (w), 2936 (w), 2870 (w), 2835 (w), 1609 (m), 1568 (w), 1514 (s), 1494 (s), 1464 (m), 1440 (m), 1380 (w), 1285 (s), 1248 (s), 1223 (m), 1181 (m), 1112 (w), 1037 (s), 867 (w), 836 (m), 795 (m)

### Example 16

### Synthesis of 4-(2-Chloro-4-methoxyphenyl)-5-isobutyl-1,2-bis(4-methoxyphenyl)-1H-pyrrole

### a) 3-(2-Chloro-4-methoxyphenyl)-1-(4-methoxyphenyl)-6-methyl-1-heptane-1,4-dione

1-(2-Chloro-4-methoxyphenyl)-3-(4-methoxyphenyl)propenone (Example 13, step a)) (1.50 g, 4.95 mmol) was refluxed with 3-methylbutyraldehyde (1.61 mL, 14.86 mmol), 3-benzyl-5-(2-hydroxyethyl)-4-methylthiazoliumchloride (401 mg, 1.49 mmol) and triethylamine (3.51 mL, 25.18 mmol) for 48 h to afford 3-(2-Chloro-4-methoxyphenyl)-1-(4-methoxyphenyl)-6-methyl-1-heptane-1 ,4-dione
C₂₂H₂₅ClO₄ (388.88)
yellow oil
**Yield:** 1.40 mmol (543 mg), 28%
**Column chromatography:** silicea gel; ligroine/diethyl ether 2:1
**¹H-NMR** [CDCl3]: **δ** = 7.94 (d, 2H, J = 8.8, Ar*H*), 7.11 (d, 1H, J = 8.7, Ar*H*), 6.99 (d, 1H, J = 2.7, Ar*H*), 6.91 (d, 2H, J = 8.9, Ar*H*), 6.79 (dd, 2H, J = 2.7, J = 8.7, Ar*H*), 4.83 (dd, 1H, J = 3.6, J = 10.0, *CH₂CH),* 3.91 (dd, 1H, J = 10.0, J = 17.8, CH₂C*H*), 3.83 (s, 3H, OC*H*₃), 3.79 (s, 3H, *OCH₃),* 3.02 (dd, 1H, J = 3.6, J = 17.8, *CH₂CH),* 2.52 (dd, 1H, J = 5.8, J = 16.5, CH₂CH(CH₃)₂), 2.29 (dd, 1H, J = 7.6, J = 16.5, CH₂CH(CH₃)₂), 2.16 (m, 1H, CH₂CH(CH₃)₂), 0.93 (d, 3H, J = 6.6, CH₂CH(CH₃)₂), 0.77 (d, 3H, J = 6.6, CH₂CH(CH₃)₂)
**MS** (EI, 250°C): m/z (%) = 388 [M]⁺' (10), 304 (5), 269 (36), 135 (100), 85 (9), 69, 57 (20), 43 (11)
**IR** (film on KBr, cm⁻¹): 3069 (w), 3005 (w), 2957 (w), 2871 (w), 2840 (w), 1714 (s), 1675 (s), 1601 (s), 1575 (m), 1510 (m), 1501 (s), 1464 (m), 1441 (w), 1420 (w), 1400 (w), 1363 (m), 1306 (w), 1287 (w), 1257 (s), 1237 (m), 1170 (s), 1143 (w), 1112 (w), 1083 (w), 1037 (s), 988 (w), 950 (w), 879 (w), 841 (m)

### b) 4-(2-Chloro-4-methoxyphenyl)-5-isobutyl-1,2-bis(4-methoxyphenyl)-1H-pyrrole

The reaction product of step a) (530 mg, 1.36 mmol) was reacted with 4-methoxyphenylamin (1.09 g, 8.86 mmol) to afford 4-(2-Chloro-4-methoxyphenyl)-5-isobutyl-1,2-bis(4-methoxyphenyl)-1*H*-pyrrole
C₂₉H₃₀ClNO₃ (476.01)
yellow solid, mp: 90°C
**Yield:** 382 µmol (182 mg), 28%
**¹H-NMR** [(D⁶)DMSO]: **δ** = 7.32 (d, 1H, J = 8.6, Ar*H*), 7.16 (d, 2H, J = 8.8, Ar*H*), 7.10 (d, 1H, J = 2.6, Ar*H*), 6.98 (d, 4H, J = 8.8, Ar*H*), 6.95 (dd, 1H, J = 2.6, J = 8.6, Ar*H*), 6.74 (d, 2H, J = 8.8, Ar*H*), 6.24 (s, 1H, 4-H), 3.80 (s, 3H, OCH₃), 3.77 (s, 3H, OCH₃), 3.68 (s, 3H, OCH₃), 2.31 (d, 2H, J = 7.3, CH₂CH(CH₃)₂), 1.14 (m, 1H, CH₂C*H*(CH₃)₂), 0.46 (d, 6H, J = 6.6, CH₂CH(CH₃)₂)
**MS** (El, 200°C): m/z (%) = 475 [M]^{+**·**} (34), 333 (100), 330 (46), 169 (17), 135 (12)
**IR** (KBr, cm⁻¹): 3000 (w), 2954 (w), 2867 (w), 1607 (m), 1569 (m), 1511 (s), 1495 (s), 1462 (m), 1443 (w), 1375 (w), 1287 (m), 1248 (s), 1178 (m), 1106 (w), 1035 (m), 835 (m), 800 (w)

### Example 17

### Synthesis of 4-(2-Fluoro-4-methoxyphenyl)-1,2-bis(4-methoxyphenyl)-5-propyl-1H-pyrrole

### a) 3-(2-Fluoro-4-methoxyphenyl)-1-(4-methoxyphenyl)propenone

1-(4-methoxyphenyl)ethanone (7.00 g, 46.61 mmol) was reacted with KOH (3.92 g, 69.92 mmol) and 2-fluoro-4-methoxybenzaldehyde (7.90 g, 51.28 mmol) according to the general experimental procedure described above to afford 3-(2-Fluoro-4-methoxyphenyl)-1-(4-methoxyphenyl)propenone.
C₁₇H₁₅FO₃ (286.30)
white solid, mp.: 92°C
**Yield:** 20.71 mmol (5.93 g), 44%
**¹H-NMR** [(D₆)DMSO]: δ = 8.14 (d, 2H, J = 8.9, Ar*H*), 8.06 (t, 1H, J = 8.9, Ar*H*), 7.86 (d, 1H, J = 15.6, CH), 7.76 (d, 1H, J = 15.6, CH), 7.09 (d, 2H, J = 8.8, Ar*H*), 6.96 (dd, 1H, J = 2.5, J = 12.9, Ar*H*), 6.90 (dd, 1H, J = 2.5, J = 8.8, Ar*H*), 3.89 (s, 3H, OCH₃), 3.89 (s, 3H, OCH₃)
**MS** (EI, 100°C): m/z (%) = 286 [M]^{+**·**} (100), 271 (24), 267 (19), 255 (42), 151 (9), 135 (51), 77(17)
**IR** (KBr, cm-¹): 3066 (w), 3017 (w), 2942 (w), 2921 (w), 2842 (w), 1653 (m), 1601 (s), 1571 (s), 1503 (m), 1469 (w), 1442 (m), 1347 (w), 1324 (w), 1300 (m), 1268 (s), 1224 (s), 1169 (m), 1088 (w), 1024 (m), 983 (w), 952 (w), 834 (m), 813 (m), 751 (w)

### b) 3-(2-Fluoro-4-methoxyphenyl)-1-(4-methoxyphenyl)heptane-1,4-dione

The reaction product of step a) (1.50 g, 5.24 mmol) was refluxed with butyraldehyde (1.42 mL, 15.72 mmol), 3-benzyl-5-(2-hydroxyethyl)-4-methylthiazoliumchloride (424 mg, 1.57 mmol) and triethylamine (2.92 ml, 20.96 mmol) for 72 h to afford 3-(2-Fluoro-4-methoxyphenyl)-1-(4-methoxyphenyl)heptane-1,4-dione.
C₂₁H₂₃FO₄ (358.40)
brown oil
**Yield:** 1.14 mmol (410 mg), 22%
**Column chromatography:** silicea gel; ligroine/diethyl ether 2:1
**¹H-NMR** [CDCl₃]: δ = 7.94 (d, 2H, J = 8.9, Ar*H*), 7.11 (m, 1H, Ar*H*), 6.90 (d, 2H, J = 8.9, Ar*H*), 6.65 (m, 2H, Ar*H*), 4.64 (dd, 1H, J = 3.9, J = 9.8, *CH₂CH),* 3.97 (dd, 1H, J = 9.9, J = 17.8, CH₂C*H*), 3.86 (s, 3H, OC*H*₃), 3.79 (s, 3H, OC*H*₃), 3.79 (dd, 1H, J = 3.9, J = 17.7, *CH₂CH),* 2.46 (ddd, 1H, J = 10.9, J = 8.3, J = 2.0, CH₂CH₂CH₃), 2.38 (ddd, 1H, J = 11.0, J = 8.4, J = 2.0, CH₂CH₂CH₃), 1.74 - 1.42 (m, 2H, CH₂CH₂CH₃), 0.82 (t, 3H, J = 7.4, CH₂CH₂CH₃)
**MS** (EI, 80°C): m/z (%) = 358 [M]^{+**.**} (14), 288 (12), 135 (100)
**IR** (film on KBr, cm⁻¹): 3073 (w), 3962 (m), 2935 (m), 2874 (w), 1712 (s), 1675 (s), 1601 (s), 1577 (w), 1509 (s), 1463 (m), 1420 (w), 1362 (w), 1310 (w), 1287 (m), 1260 (s), 1170 (s), 1156 (w), 1120 (m), 1029 (s), 950 (w), 836 (w)

### c) 4-(2-Fluoro-4-methoxyphenyl)-1,2-bis(4-methoxyphenyl)-5-propyl-1H-pyrrole

The reaction product of step b) (430 mg, 1.20 mmol) was reacted with 4-methoxyphenylamine (960 mg, 7.80 mmol) to afford 4-(2-Fluoro-4-methoxyphenyl)-1,2-bis(4-methoxyphenyl)-5-propyl-1*H*-pyrrole.
C₂₈H₂₈FN0₃ (445.53)
brown solid, mp: 80°C
**Yield:** 364 µmol) (162 mg), 30%
**¹H-NMR** [(D₆)DMSO]: δ = 7.31 (d, 1H, J = 8.7, Ar*H*), 7.17 (d, 2H, J = 8.6, Ar*H*), 6.98 (d, H, J = 8.8, Ar*H*), 6.94 (d, 2H, J = 8.8, Ar*H*), 6.89 (dd, 1H, J = 2.3, J = 12.4, Ar*H*), 6.83 (dd, 1H, J = 2.4, J = 8.5, Ar*H*), 6.72 (d, 2H, J = 8.6, Ar*H*), 6.25 (s, 1H, 4-H), 3.80 (s, 3H, OC*H*₃), 3.79 (s, 3H, OC*H*₃), 3.68 (s, 3H, OC*H*₃2.40 (t, 2H, J = 7.6, C*H*₂CH₂CH₃), 1.11 (m, 2H, CH₂CH₂CH₃) 0.54 (t, 3H, J = 7.3, C*H*₂CH₂CH₃)
**MS** (El, 200°C): m/z (%) = 445 [M]^{+**·**} (69), 416 (100), 311 (10), 223 (6), 135 (16)
**IR** (KBr, cm⁻¹): 3037 (w), 2959 (w), 2836 (w), 1626 (m), 1577 (m), 1514 (s), 1503 (s), 1465 (m), 1441 (m), 1380 (w), 1289 (s), 1248 (s), 1181 (m), 1147 (m), 1122 (m), 1031 (m), 835 (m)

### Example 18

### Synthesis of 4-(2-Chloro-4-hydroxyphenyl)-1,2-bis(4-hydroxyphenyl)-5-methyl-1H-pyrrole

4-(2-Chloro-4-methoxyphenyl)-1,2-bis(4-methoxyphenyl)-5-methyl-1*H*-pyrrole (Example 13) (70 mg, 161 µmol) was reacted with BBr₃ (76 µL, 807 µmol) according to the general experimental procedure described above to afford 4-(2-Chloro-4-hydroxyphenyl)-1,2-bis(4-hydroxyphenyl)-5-methyl-1*H*-pyrrole.
C₂₃H₁₈ClNO₃ (391.85)
yellow solid, mp: 140°C
**Yield:** 138 µmol (54 mg), 86%
**Column chromatography:** silicea gel; CH2C12/MeOH 95:5
**¹H-NMR** [(D₆)DMSO]: δ = 9.77 (s, 1H, OH), 9.68 (s, 1H, OH), 9.29 (s, 1H, OH), 7.19 (d, 1H, J = 8.3, Ar*H*), 7.01 (d, 2H, J = 8.6, Ar*H*), 6.89 (d, 1H, J = 2.4, Ar*H*), 6.87 (d, 2H, J = 8.7, Ar*H*), 6.78 (d, 2H, J = 8.4, Ar*H*), 6.75 (dd, 1H, J = 2.3, J = 8.2, Ar*H*), 6.55 (d, 2H, J = 8.5, Ar*H*), 6.19 (s, 1H, 4-*H*), 1.88 (s, 3H, C*H*₃)
**MS** (El, 260°C): m/z (%) = 391 [M]^{+·} (100), 257 (6), 223 (25)
**IR** (KBr, cm⁻¹): 3400 (s), 3037 (w), 2973 (w), 1609 (m), 1573 (m), 1516 (s), 1496 (m), 1443 (m), 1378 (m), 1264 (m), 1223 (m), 1174 (m), 1104 (w), 1055 (w), 895 (w), 837 (m)
CHN (%): Calc.: C 70.50 H 4.63 N 3.57, Found: C 69.97 H 4.56 N 3.99

### Example 19

### Synthesis of 4-(2-Chloro-4-hydroxyphenyl)-5-ethyl-1,2-bis(4-hydroxyphenyl)-1H-pyrrole

4-(2-Chloro-4-methoxyphenyl)-5-ethyl-1,2-bis(4-methoxyphenyl)-1*H*-pyrrole (Example 14) (80 mg, 179 µmol) was reacted with BBr₃ (84 µL, 893 µmol) according to the general experimental procedure described above to afford 4-(2-Chloro-4-hydroxyphenyl)-5-ethyl-1,2-bis(4-hydroxyphenyl)-1*H*-pyrrole.
C₂₄H₂₀ClNO₃ (405.87)
yellow solid, mp: 145°C
**Yield:** 153 µmol (62 mg), 85%
**Column chromatography:** silicea gel; CH2Cl2/MeOH 95:5
**¹H-NMR** [(D₆)DMSO]: δ = 9.78 (s, 1H, OH), 9.70 (s, 1H, OH), 9.28 (s, 1H, OH), 7.18 (d, 1H, J = 8.3, Ar*H*), 7.02 (d, 2H, J = 8.5, Ar*H*), 6.90 (d, 1H, J = 2.4, Ar*H*), 6.84 (d, 2H, J = 8.5, Ar*H*), 6.78 (d, 2H, J = 8.4, Ar*H*), 6.74 (dd, 1H, J = 2.3, J = 8.2, Ar*H*), 6.54 (d, 2H, J = 8.5, Ar*H*), 6.130 (s, 1H, 4-H), 3.82 (s, 3H, OC*H*₃), 3.77 (s, 3H, OC*H*₃), 3.66 (s, 3H, OC*H*₃), 2.39 (q, 2H, J = 7.4, C*H*₂CH₃), 0.67 (t, 3H, J = 7.4, CH₂C*H*₃)
**MS** (EI, 200°C): m/z (%) = 405 [M]^{+**.**} (100), 392 (44), 390 (72), 377 (36), 355 (26), 203 [M]⁺⁺ (5)
**IR** (KBr, cm⁻¹): 3421 (s), 3068 (w), 2969 (w), 2925 (w), 1611 (m), 1513 (s), 1495 (m), 1453 (m), 1383 (s), 1267 (m), 1223 (m), 1175 (w), 1105 (w), 839 (w), 747 (w)
CHN (%): Calc.: C 71.02 H 4.97 N 3.45, Found: C 70.36 H 4.68 N 3.42

### Example 20

### Synthesis of 4-(2-Chloro-4-hydroxyphenyl)-1,2-bis(4-hydroxyphenyl)-5-propyl-1H-pyrrole

4-(2-Chloro-4-methoxyphenyl)-1,2-bis(4-methoxyphenyl)-5-propyl-1*H*-pyrrole (Example 15) (101 mg, 219 µmol) was reacted with BBr₃ (103 µL, 1.09 mmol) according to the general experimental procedure described above to afford 4-(2-Chloro-4-hydroxyphenyl)-1,2-bis(4-hydroxyphenyl)-5-propyl-1*H*-pyrrole.
C₂₅H₂₂ClN0₃ (419.90)
white solid, mp: 144°C
**Yield:** 202 µmol (85 mg), 92%
**Column chromatography:** silicea gel; CH₂Cl₂/MeOH 95:5
¹H-NMR [(D₆)DMSO]: δ = 9.74 (s, 1H, OH), 9.66 (s, 1H, OH), 9.25 (s, 1H, OH), 7.18 (d, 1H, J = 8.3, Ar*H*), 7.02 (d, 2H, J = 8.7, Ar*H*), 6.89 (d, 1H, J = 2.4, Ar*H*), 6.85 (d, 2H, J = 8.6, Ar*H*), 6.78 (d, 2H, J = 8.6, Ar*H*), 6.74 (dd, 1H, J = 2.3, J = 8.3, Ar*H*), 6.52 (d, 2H, J = 8.6, Ar*H*) 6.13 (s, 1H, 4-H), 2.52 (m + (D₆)DMSO, 2H, CH₂CH₂CH₃), 1.02 (m, 2H, CH₂CH₂CH₃) 0.51 (t, 3H, J = 7.3, CH₂CH₂CH₃)
**MS** (EI, 200°C): m/z (%) = 419 [M]^{+**.**} (70), 406 (10), 390 (100), 377 (38), 355 (22), 223 (14), 212 (14), 196 (3)
**IR** (KBr, cm⁻¹): 3417 (s), 3057 (w), 2958 (w), 2930 (w), 1602 (m), 1567 (w), 1514 (s), 1494 (m), 1449 (m), 1405 (w), 1378 (w), 1253 (m), 1219 (m), 1176 (m), 1098 (w), 836 (w)
**CHN** (%): Calc.: C 71.51 H 5.28 N 3.34, Found: C 71.85 H 5.08 N 3.69

### Example 21

### Synthesis of 4-(2-Chloro-4-hydroxyphenyl)-5-isobutyl-1,2-bis(4-hydroxyphenyl)-1H-pyrrole

4-(2-Chloro-4-methoxyphenyl)-5-isobutyl-1,2-bis(4-methoxyphenyl)-1*H*-pyrrole (Example 16) (104 mg, 218 µmol) was reacted with BBr₃ (108 µL, 1.15 µmol) according to the general experimental procedure described above to afford 4-(2-Chloro-4-hydroxyphenyl)-5-isobutyl-1,2-bis(4-hydroxyphenyl)-1*H*-pyrrole.
C₂₆H₂₄ClNO₃ (433.93)
yellow solid, mp: 134°C
**Yield:** 175 µmol (76 mg), 80%
**Column chromatography:** silicea gel; ligroine/ethyl acetate 1:1
**¹H-NMR** [(D₆)DMSO]: δ = 9.74 (s, 1H, OH), 9.70 (s, 1H, OH), 9.28 (s, 1H, OH), 7.18 (d, 1H, J = 8.4, Ar*H*), 6.98 (d, 2H, J = 8.6, Ar*H*), 6.85 (d, 1H, J = 2.4, Ar*H*), 6.84 (d, 2H, J = 8.6, Ar*H*), 6.77 (d, 2H, J = 8.6, Ar*H*), 6.75 (dd, 1H, J = 2.5, J = 8.4, Ar*H*), 6.54 (d, 2H, J = 8.7, Ar*H*), 6.13 (s, 1H, 4-H), 2.29 (d, 2H, J = 7.3, CH₂CH(CH₃)₂), 1.18 (m, 1H, CH₂CH(CH₃)₂), 0.46 (d, 6H, J = 6.6, CH(CH₃)₂)
**MS** (El, 260°C): m/z (%) = 433 [M]⁺' (46), 390 (100), 355 (16), 354 (13)
**IR** (KBr, cm⁻¹): 3409 (s), 3037 (w), 2955 (w), 2929 (w), 1611 (m), 1569 (w), 1514 (s), 1496 (m), 1444 (m), 1378 (w), 1339 (w), 1261 (m), 1221 (m), 1174 (m), 1100 (w), 838 (w)
**CHN** (%): Calc.: C 71.95 H 5.57 N 3.23, Found: C 71.02 H 5.78 N 2.93

### Example 22

### Synthesis of 4-(2-Fluoro-4-hydroxyphenyl)-1,2-bis(4-hydroxyphenyl)-5-propyl-1H-pyrrole

4-(2-Fluoro-4-methoxyphenyl)-1,2-bis(4-methoxyphenyl)-5-propyl-1*H-*pyrrole (Example 17) (96 mg, 216 µmol) was reacted with BBr₃ (108 µL, 1.15 µmol) according to the general experimental procedure described above to afford 4-(2-Fluoro-4-hydroxyphenyl)-1,2-bis(4-hydroxyphenyl)-5-propyl-1*H*-pyrrole.
C₂₅H₂₃FNO₃ (403.45)
brown solid, mp: 92°C
Yield: 216 µmol (87 mg), 72%
**¹H-NMR** [(D₆)DMSO]: δ = 9.70 (s, 1H, OH), 9.63 (s, 1H, OH), 9.21 (s, 1H, OH), 7.29 (d, 1H, J = 8.7, Ar*H*), 7.18 (d, 2H, J = 8.5 Ar*H*), 6.96 (d, H, J = 8.7 Ar*H*), 6.94 (d, 2H, J = 8.8, Ar*H*), 6.93 (dd, 1H, J = 2.3, J = 12.4, Ar*H*), 6.81 (dd, 1H, J = 2.4, J = 8.5, Ar*H*), 6.70 (d, 2H, J = 8.7, Ar*H*), 6.22 (s, 1H, 4-H), 2.44 (t, 2H, J = 7.6, CH₂CH₂CH₃), 1.11 (m, 2H, CH₂CH₂CH₃) 0.51 (t, 3H, J = 7.4, CH₂CH₂CH₃)
**MS** (EI, 220°C): m/z (%) = 403 [M]^{+.} (74), 374 (100), 202 (7), 121 (17)
**IR** (KBr, cm⁻¹): 3400 (s), 2959 (w), 2870 (w), 1626 (m), 1576 (m), 1513 (s), 1503 (s), 1287 (m), 1246 (s), 1181 (m), 1121 (m), 1031 (m), 835 (m)
CHN (%): Calc.: C 74.43 H 5.50 N 3.47, Found: C 74.02 H 5.88 N 3.13

### Determination of the biological activity of the compounds of formula I

### a) COX inhibition

### Materials and Methods

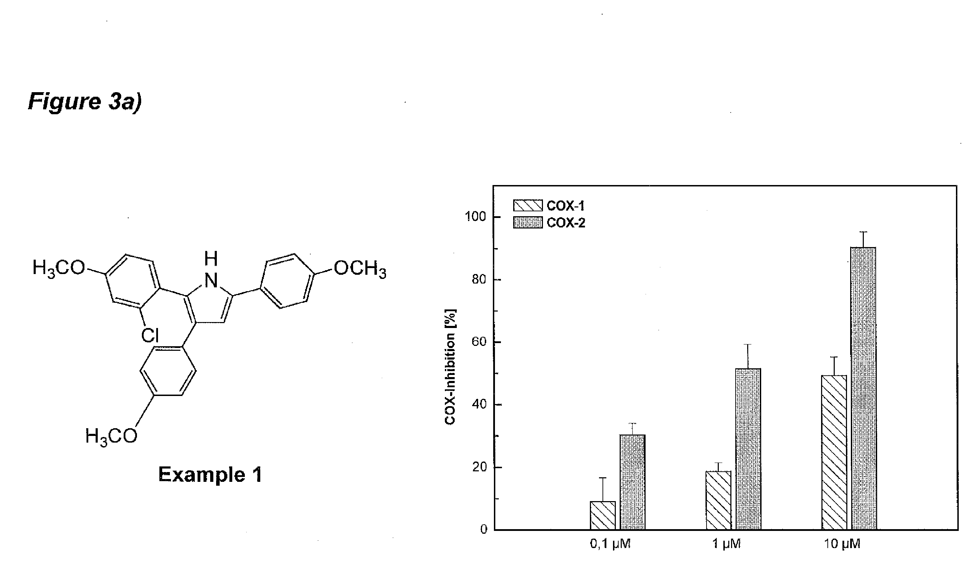

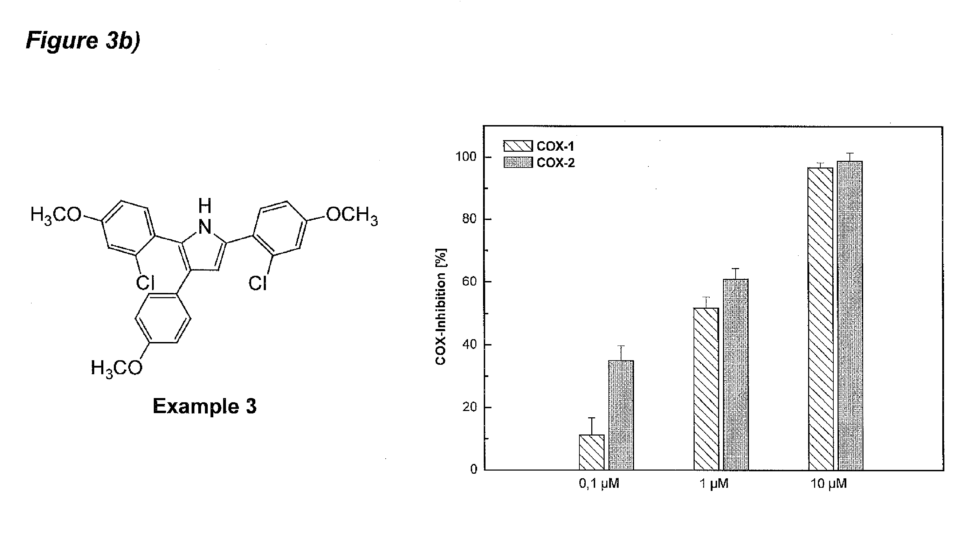

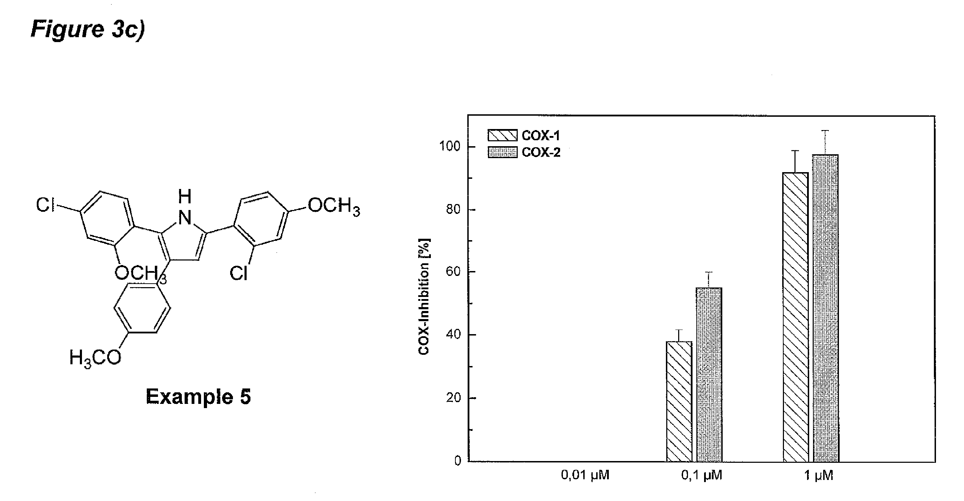

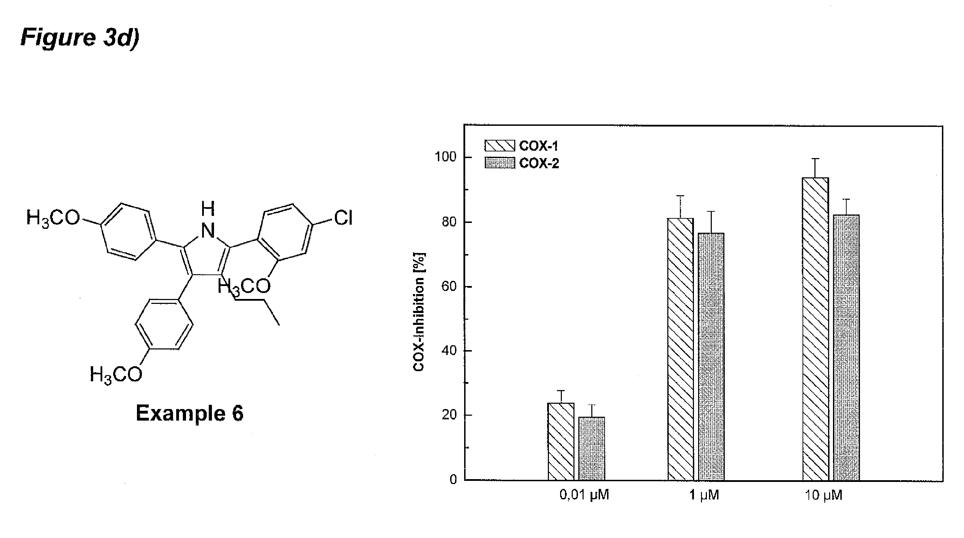

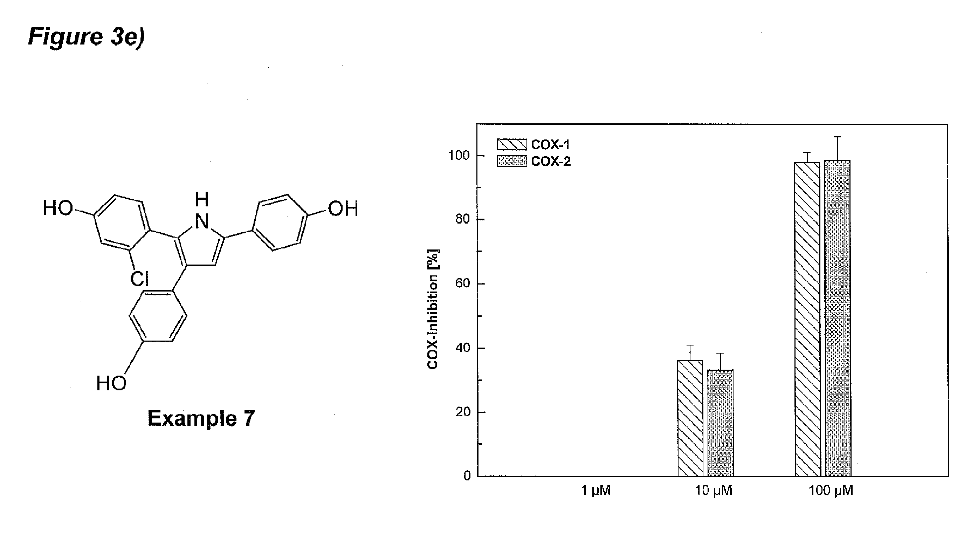

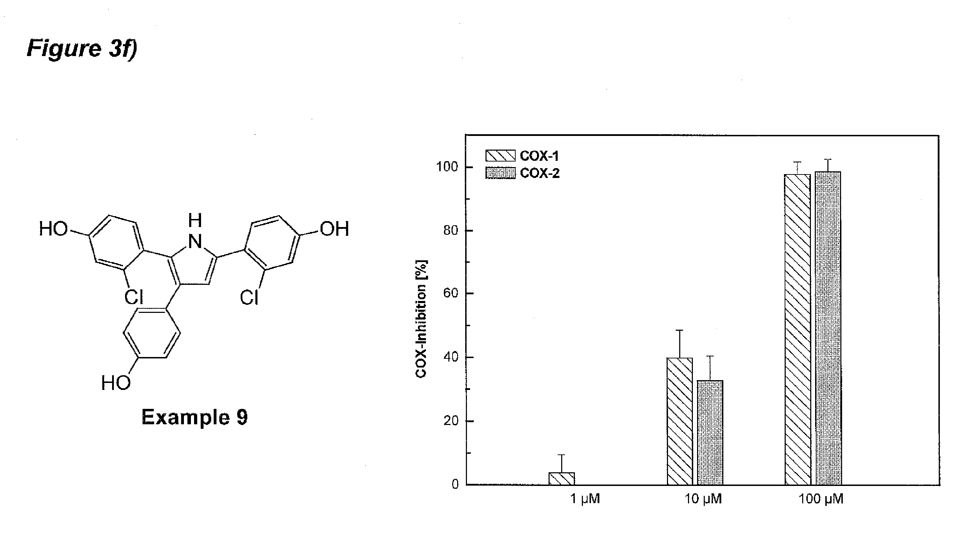

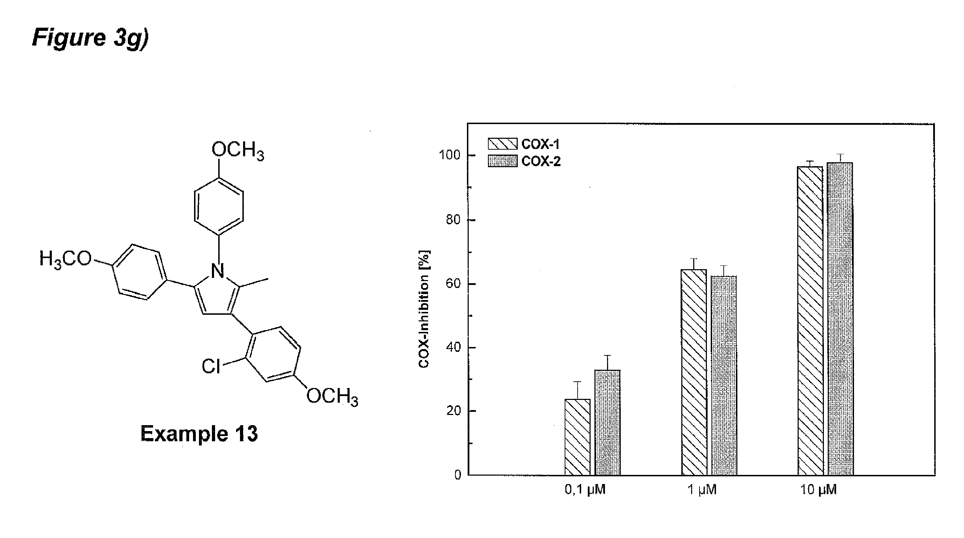

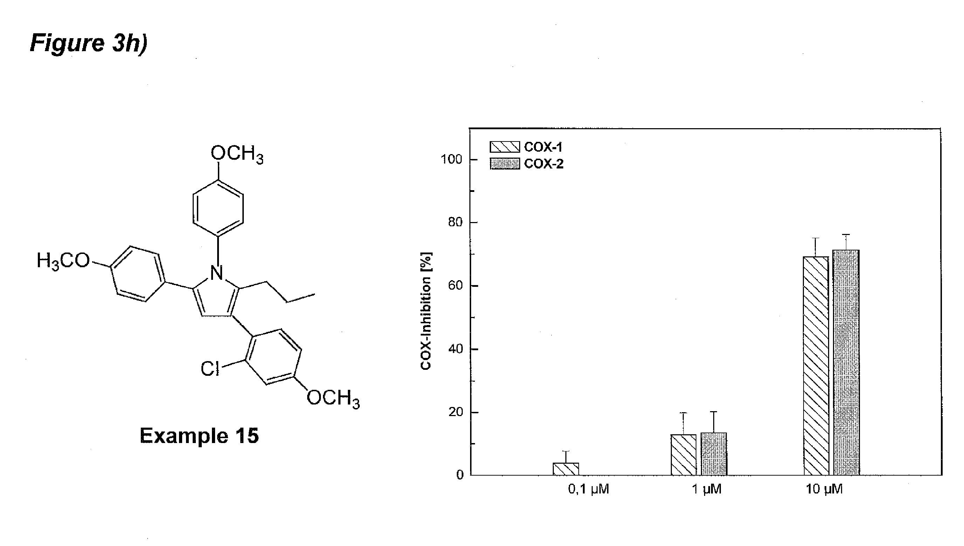

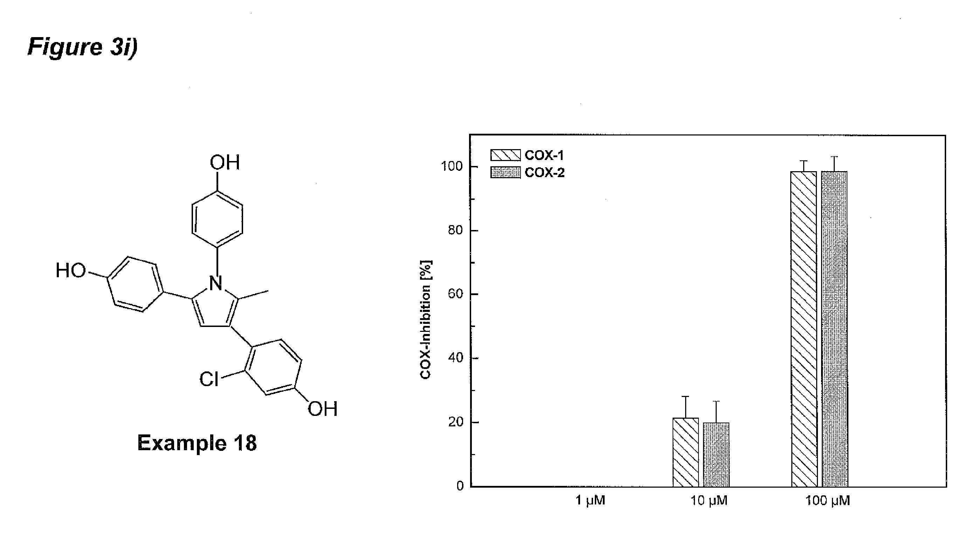

The effect of the compounds on COX-1 and COX-2 was determined by quantifying prostaglandin E₂ (PGE₂) using a COX Inhibitor Screening Kit Catalog No 560131) from Cayman Chemicals, Ann Arbor Michigan USA. Reaction mixtures were prepared in 100 mM TrisHCl buffer, pH 8.0 containing 1 µM heme and COX-1 (ovine) or COX-2 (human recombinant) and preincubated for 10 min in a waterbath (37 °C). The reaction was initiated by the addition of 10 µL arachidonic acid. After 2 min the reaction mixture was terminated by adding 1M HCl and PGE₂ was quantified by an ELISA method. The test compounds were dissolves in DMSO and diluted to the desired concentration with 100 mM potassium phosphate buffer (pH 7.4). After this the solutions were transferred to a 96-well plate coated with a mouse anti-rabbit IgG, the tracer prostaglandin esterase and primary antibody (mouse anti PGE₂) were added. Then the plate was incubated for 18 h at room temperature. After removing the reaction mixture, the wells were washed with 10 mM potassium phosphate buffer containing 0.05% Tween 20. Finally Ellman's reagent (200 µL) was added to each well and plate was incubated at room temperature until the control wells yielded an OD= 0.3-0.8 at 410 nm (45-60 min). A standard curve with PGE₂ was generated from the same plate, in order to quantify the PGE₂ levels produced in the presence of the test compounds. The results were expressed as a percentage to a solvent treated control sample. Exemplary compounds of formula I are shown in ***Figures 3a)* to *3i).***

### b) Cytotoxic effects

### Materials and Methods

### Time dependent test

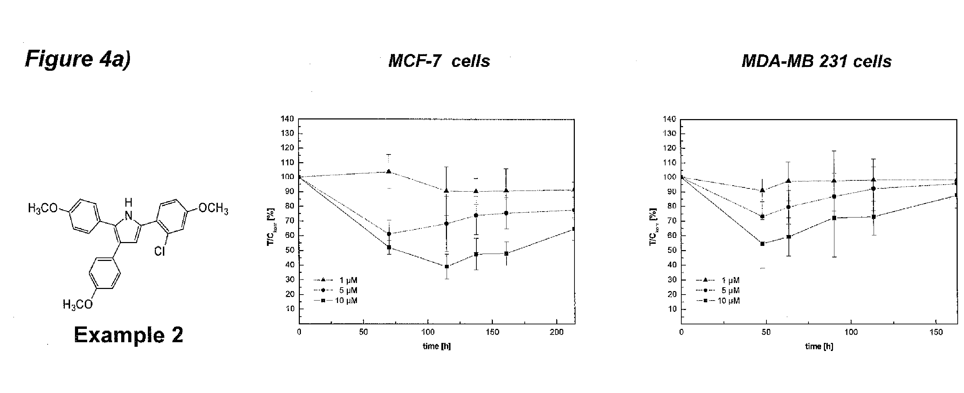

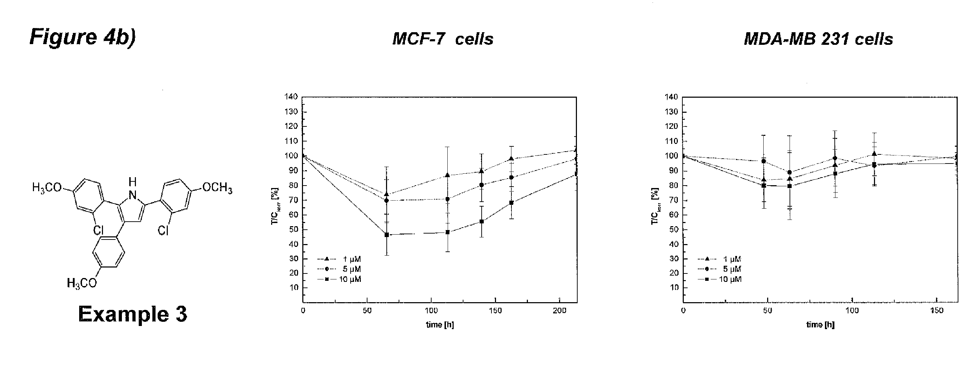

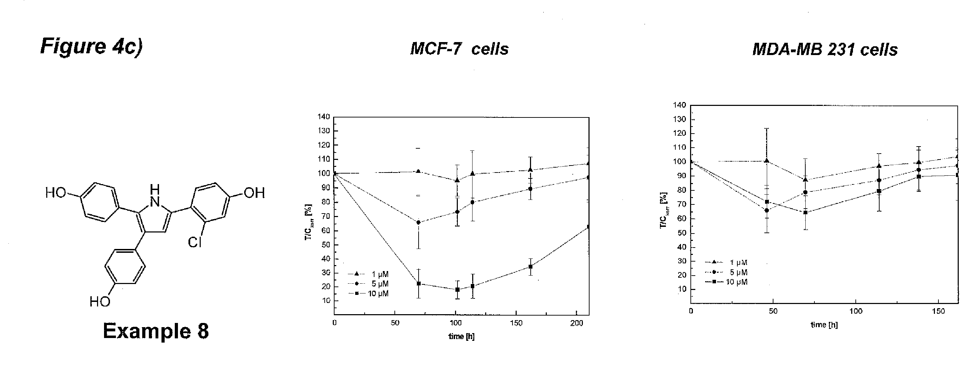

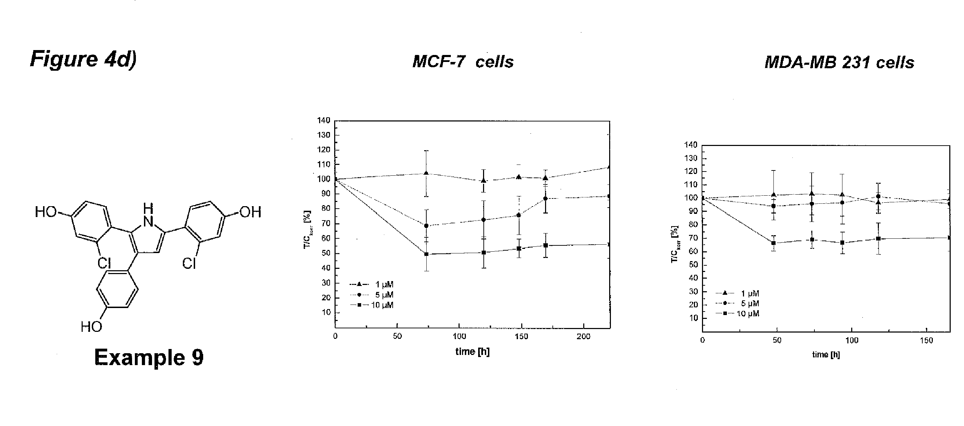

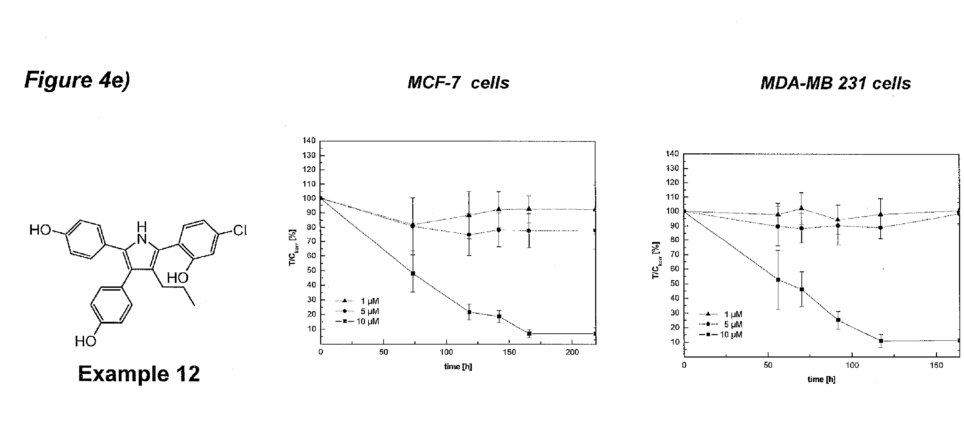

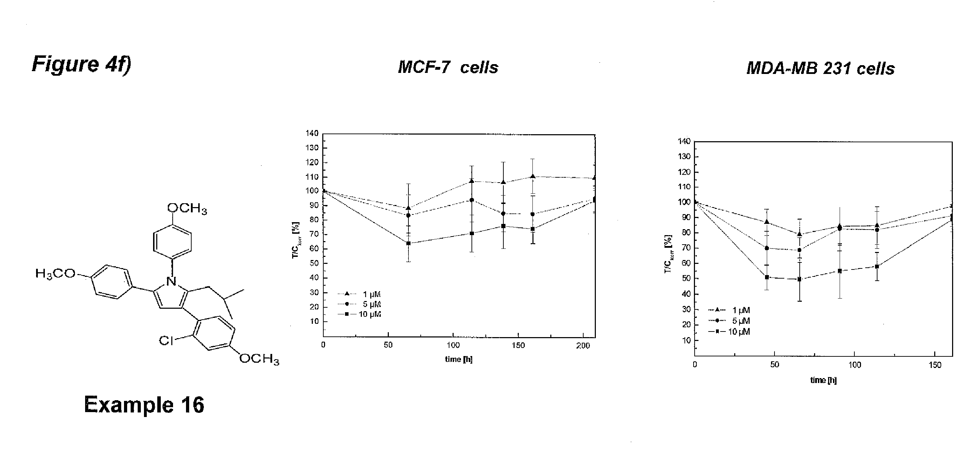

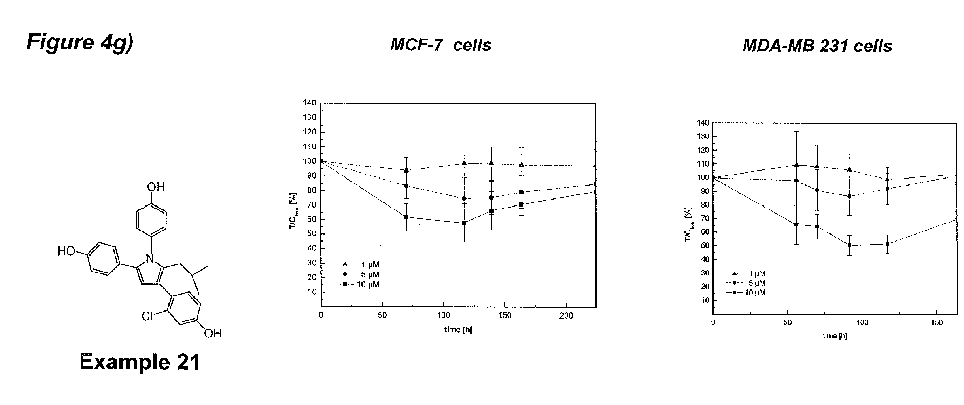

The cytotoxic assay in estrogen receptor positive MCF-7 cells has been described previously by Ruenitz, P.C. et al. (1997) J. Steroid Biochem. Mol. Biol. 63, 203-209). Fiation, staining and quantitation of the cells were carried out according to Gillies (Gillies, R.J. et al. (1986) Anal. Biochem. 159, 109-113) and Kueng (Kueng, W. et al. (1989) Anal. Biochem. 182, 16-19). Moreover the test was carried out with estrogen receptor negative MD-MB 231 cells. MCF-7 cells (MDA-MB 231 cells) from an almost confluent monolayer were harvest by trypsinization and suspended to approximately 7 x 10³ cells/mL (1 x 10³ cells/mL). At the beginning of the experiment, the cell suspension was transferred to 96-well microplates (100 µL/well). After cultivating them for 3 days at growing conditions the medium was removed and replaced by one containing the test compounds. Control wells (16/plate) were contained 0.1% of DMSO, which was used for the preparation of the stock solutions. The initial cell density was determined by addition of glutaric dialdehyde (1 % in PBS; 100 µL/well). After incubation for 3-7 days, the medium was removed, and glutaric dialdehyde (1 % in PBS; 100 µL/well) was added for fixation. After 15 min, the solution of the aldehyde was decanted and 180 µL PBS/well added. The plates were stored at 4°C until staining. Cells were stained by treating them for 25 min with 100 µL of an aqueous solution of crystal violet (0.02%). After decanting cells were washed several times with water to remove the adherent dye. After addition of 180 µL of ethanol (70%), plates were gently shaken for 4 h. Optical density of each well was measured in a microplate autoreader at 590 nm. Results are shown in ***Figures 4a)* to *4g).***

### Concentration dependent test (IC₅₀)

MCF-7 cells (MDA-MB 231 cells) from an almost confluent monolayer were harvested by trypsinization and suspended to approximately 10 × 10³ cells/mL (5 x 10³ cells/mL). After cultivating them for 3 days at growing conditions the medium was removed and replaced by one containing the test compounds (control wells 8/plate). After incubation of 72 h (48 h) the medium was removed and the test was continued as descried above. Results are shown in ***Table 1**.*

**Table 1**

| **IC₅₀-values** | | |
|---|---|---|
| **compound** | **MCF-7 cells** **IC₅₀ [µM]** | **MDA-MB 231 cells** **IC₅₀ [µM]** |
| **Example 1** | 17.7 | 27.4 |
| **Example 2** | 9.5 | 10.3 |
| **Example 7** | 16.4 | 13.5 |
| **Example 9** | 7.6 | 13.4 |
| **Example 10** | 9.2 | 18.8 |
| **Example 11** | 10.5 | 14.0 |
| **Example 12** | 8.1 | 6.5 |
| **Example 13** | 23.6 | 20.3 |
| **Example 16** | 18.0 | 15.2 |
| **Example 17** | 17.8 | 18.4 |
| **Example 18** | 23.4 | 17.4 |
| **Example 19** | 17.8 | 18.4 |
| **Example 20** | 19.5 | 16.2 |
| **Example 21** | 15.2 | 16.1 |

### c) Estrogenic effects: Determination of the relative estrogen receptor binding affinities (RBA) in the whole cell assay and on the isolated recombinant estrogen receptor α (ERα).

ER binding affinity is measured in competitive binding assays compared to estradiol. Binding affinity is expressed as a relative binding affinity (RBA) in percent compared to estradiol which is assigned an affinity of 100%. Substantial affinity for ER is indicated by an RBA of about 0,1% or more. Good affinity binding to ER is indicated by an RBA of about 1%- to about 10%. High affinity binding to ER is indicated by an RBA of about 10% or higher.

### Materials and Methods

### Whole Cell Assay (WCA)

MCF-7 cells were plated in 24-well dishes (4 x 10⁴ cells/well) and cultured for 48 h. After the incubation the medium was replaced by test compounds diluted in serum free medium containing [³H]17β-Estradiol ([³H]E2). Additional wells of each test compound concentration were filled with 500-fold excess of unlabeled E2 for nonspecific binding measurements. After 1 h incubation (37°C) the medium was removed and monolayer washed three times with an ice-cold saline solution. Bond E2 was then extracted from the monolayer with 1 mL ethanol at room temperature (20 min of incubation) and aliquots of 200 µL of ethanolic extracts were transferred to scintillation vials containing 3.8 mL scintillator Ecoscint H (National Diagnostic, Atlanta) for radioactivity counting. All measurements were performed in duplicate. Specific [³H]E2 incorporation into the cells was calculated from the difference in incorporated radioactivity after incubation in the absence or presence of an excess of unlabeled E2. The results (relative radioactivity) were expressed as a percentage to a solvent treated control sample. Results are shown in ***Table 2**.*

### Recombinant ERa (HAP Assay)

An ethanolic solution (250 fmol/µL) of recombinant human estrogen receptor (hERα) (Calbiochem) was diluted with Tris-HCl buffer pH 8.0 (1:100). This hERα preparations adsorbed on hydroxylapatite pellets (prepared in 10 mmol/L Tris HCl buffer pH 8.0) which were labeled with increasing concentrations of the test compound (10⁻¹⁰ - 10⁻⁵ M) and [³H]E2 (0.1 %) in presence or absence of 200-fold excess of unlabeled E2 and were incubated overnight at 4°C. Bond [³H]E2 were then successively extracted with ethanol and aliquots of 200 µL of ethanolic extracts were transferred to scintillation vials containing 3.8 mL scintillator Ecoscint H (National Diagnostic, Atlanta) for radioactivity counting. The data interpretation was carried out as shown above. Results are shown in ***Table 2**.*

**Table 2**

| **Relative radioactivity of labeled [³H]E2 [%]** | | |
|---|---|---|
| | **RBA-value** | **RBA-value** |
| **compound** | **MCF-7 cells (WCA); 10⁻⁶ M** | **HAP-Assay; 10⁻⁶ M** |
| **Example 7** | 35.3 ± 4.9 | 26.4 ± 8.3 |
| **Example 9** | 13.1 ± 3.2 | 19.1 ± 1.5 |
| **Example 12** | 78.1 ± 4.4 | 70.3 ± 6.1 |
| **Example 19** | 41.2 ± 8.3 | 30.1 ± 5.1 |

## Claims

1. Triphenyl modified 5-membered heterocycles of the general formula I 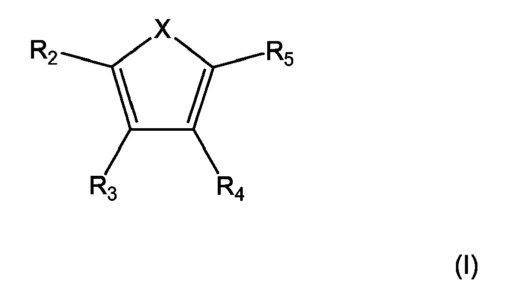 wherein
X represents NR¹, O or S
R¹ to R⁵ independently from each other represent H, alkyl, unsubstituted phenyl or phenyl substituted with hydroxy, alkoxy, alkyl, trihalomethyl and/or halogen,
wherein at least one of the three phenyl moieties is substituted with halogen or trihalomethyl
or their salts or esters
with exclusion of 2,3-diphenyl-5-(o-bromophenyl)pyrrole, 2,3-diphenyl-5-(m-iodophenyl)pyrrole, 2-(p-methoxyphenyl-3-(m-ethoxyphenyl)-5-(p-fluorophenyl) pyrrole and 2-(p-fluorophenyl)-3-(m-butylphenyl)-5-(p-trifluoromethyl-phenyl) pyrrole.

2. Compounds according to claim 1, wherein at least one of the three phenyl moieties is substituted with halogen and alkoxy, halogen and hydroxy, trihalomethyl and alkoxy, trihalomethyl and hydroxy or halogen and alkyl.

3. Compounds according to claim 1, wherein two or three phenyl moieties are substituted with alkoxy and/or hydroxy.

4. Compounds according to claim 1, wherein two or three phenyl moieties are substituted with halogen and/or trihalomethyl.

5. Compounds according to claim 1, wherein the two non-phenyl moieties of R¹ to R⁵ are both hydrogen or one is hydrogen and the other alkyl.

6. Compounds according to claim 1 being 2,3,5-triphenyl pyrroles, -furans or -thiophenes.

7. Compounds according to claim 1 having the following structure:
1) 2-(2-Chloro-4-methoxyphenyl)-3,5-bis(4-methoxyphenyl)-1*H*-pyrrole
2) 5-(2-Chloro-4-methoxyphenyl)-2,3-bis(4-methoxyphenyl)-1*H*-pyrrole
3) 2,5-Bis(2-Chloro-4-methoxyphenyl)-3-(4-methoxyphenyl)-1*H*-pyrrole
4) 2-(4-Chloro-2-methoxyphenyl)-3,5-bis(4-methoxyphenyl)-1*H*-pyrrole
5) 5-(2-Chloro-4-methoxyphenyl)-2-(4-chloro-2-methoxyphenyl)-3-(4-methoxyphenyl)-1*H*-pyrrole
6) 5-(4-Chloro-2-methoxyphenyl)-2,3-bis(4-methoxyphenyl)-4-propyl-1*H-*pyrrole
7) 2-(2-Chloro-4-hydroxyphenyl)-3,5-bis(4-hydroxyphenyl)-1*H*-pyrrole
8) 5-(2-Chloro-4-hydroxyphenyl)-2,3-bis(4-hydroxyphenyl)-1*H*-pyrrole
9) 2,5-Bis(2-Chloro-4-hydroxyphenyl)-3-(4-hydroxyphenyl)-1*H*-pyrrole
10) 2-(4-Chloro-2-hydroxyphenyl)-3,5-bis(4-hydroxyphenyl)-1*H*-pyrrole
11) 5-(2-Chloro-4-hydroxyphenyl)-2-(4-chloro-2-hydroxyphenyl)-3-(4-hydroxyphenyl)-1*H*-pyrrole
12) 5-(4-Chloro-2-hydroxyphenyl)-2,3-bis(4-hydroxyphenyl)-4-propyl-1*H-*pyrrole
13) 2,5-Bis(2-chloro-4-ethoxyphenyl)-3-(4-ethoxyphenyl)-1*H*-pyrrole
14) 2-(2-Fluoro-4-methoxyphenyl)-3,5-bis(4-methoxyphenyl)-1*H*-pyrrole
15) 2,3,5-Tris(2-chloro-4-methoxyphenyl)-1*H*-pyrrole
16) 2-(2-Chloro-4-methoxyphenyl)-5-(2-fluoro-4-methoxyphenyl)-3-(4-methoxyphenyl)-1*H*-pyrrole
17) 5-(2-Chloro-4-methylphenyl)-2,3-di-p-toluyl-1*H*-pyrrole
18) 3,5-Bis(4-methoxyphenyl)-2-(2-trifluoromethyl-4-methoxyphenyl)--1*H-*pyrrole
19) 2,5-Bis(2-chloro-4-methoxyphenyl)-3-(4-methoxyphenyl)-furan

8. Compounds according to claim 1 being 1,2,4-triphenyl pyrroles.

9. Compounds according to claim 1 having the following structure:
20) 4-(2-Chloro-4-methoxyphenyl)-1,2-bis(4-methoxyphenyl)-5-methyl-1*H-*pyrrole
21) 4-(2-Chloro-4-methoxyphenyl)-5-ethyl-1,2-bis(4-methoxyphenyl)-1*H-*pyrrole
22) 4-(2-Chloro-4-methoxyphenyl)-1,2-bis(4-methoxyphenyl)-5-propyl-1*H-*pyrrole
23) 4-(2-Chloro-4-methoxyphenyl)-5-isobutyl-1,2-bis(4-methoxyphenyl)-1*H-*pyrrole
24) 4-(2-Fluoro-4-methoxyphenyl)-1,2-bis(4-methoxyphenyl)-5-propyl-1*H-*pyrrole
25) 4-(2-Chloro-4-hydroxyphenyl)-1,2-bis(4-hydroxyphenyl)-5-methyl-1*H-*pyrrole
26) 4-(2-Chloro-4-hydroxyphenyl)-5-ethyl-1,2-bis(4-hydroxyphenyl)-1*H-*pyrrole
27) 4-(2-Chloro-4-hydroxyphenyl)-1,2-bis(4-hydroxyphenyl)-5-propyl-1*H-*pyrrole
28) 4-(2-Chloro-4-hydroxyphenyl)-5-isobutyl-1,2-bis(4-hydroxyphenyl)-1*H-*pyrrole
29) 4-(2-Fluoro-4-hydroxyphenyl)-1,2-bis(4-hydroxyphenyl)-5-propyl-1*H-*pyrrole
30) 4-(2-Chloro-4-propoxyphenyl)-1,2-bis(4-propoxyphenyl)-5-methyl-1*H-*pyrrole
31) 4-(2-Fluoro-4-methoxyphenyl)-1,2-bis(4-methoxyphenyl)-5-methyl-1*H-*pyrrole
32) 4-(2-Chloro-4-methoxyphenyl)-1,2-bis(4-methoxyphenyl)-1*H*-pyrrole
33) 1,2,4-Tris(2-chloro-4-methoxyphenyl)-1*H*-pyrrole
34) 1,2,4-Tris(2-chloro-4-methylphenyl)-1*H*-pyrrole
35) 1,2,4-Tris(2-chlorophenyl)-5-methyl-1*H-*pyrrole
36) 4-(3-Chloro-5-methoxyphenyl)-1,2-bis(4-methoxyphenyl)-5-methyl-1*H-*pyrrole
37) 4-(2-Chloro-4-methoxyphenyl)1,2-bis(4-methoxyphenyl)-3-methyl-1*H-*pyrrole

10. Use of one or more triphenyl modified 5-membered heterocycles of the general formula I 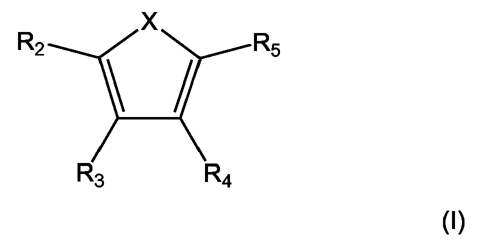 wherein
X represents NR¹, O or S
R¹ to R⁵ independently from each other represent H, alkyl, unsubstituted phenyl or phenyl substituted with hydroxy, alkoxy, alkyl, trihalomethyl and/or halogen,
wherein at least one of the three phenyl moieties is substituted with halogen or trihalomethyl
or their salts or esters
for the manufacture of a pharmaceutical composition for prevention and treatment of cancer or inflammatory diseases.

11. One or more triphenyl modified 5-membered heterocycles of the general formula I 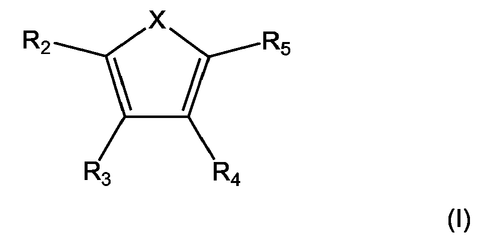 wherein
X represents NR¹, O or S
R¹ to R⁵ independently from each other represent H, alkyl, unsubstituted phenyl or phenyl substituted with hydroxy, alkoxy, alkyl, trihalomethyl and/or halogen,
wherein at least one of the three phenyl moieties is substituted with halogen or trihalomethyl
for use as anticancer agent or anti-inflammatory agent.

12. A pharmaceutical composition comprising as active substance one or more triphenyl modified 5-membered heterocycles of the general formula I 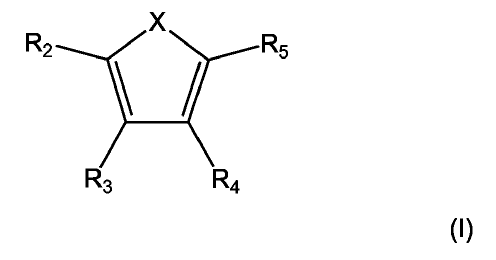 wherein
X represents NR¹, O or S
R¹ to R⁵ independently from each other represent H, alkyl, unsubstituted phenyl or phenyl substituted with hydroxy, alkoxy, alkyl, trihalomethyl and/or halogen,
wherein at least one of the three phenyl moieties is substituted with halogen or trihalomethyl
in an amount sufficient to exhibit a therapeutic or preventive effect.

13. A method for treating cancer or an inflammatory disease which comprises the step of administering to a patient suffering from that disorder a therapeutically effective amount of at least one compound of formula I.

14. Use of one or more triphenyl modified 5-membered heterocycles of the general formula I 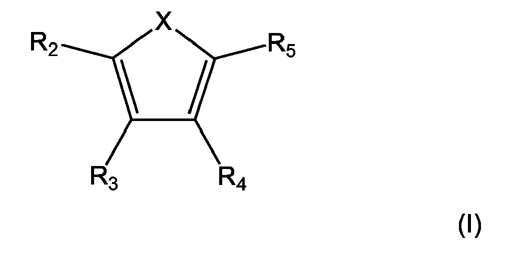 wherein
X represents NR¹, O or S
R¹ to R⁵ independently from each other represent H, alkyl, unsubstituted phenyl or phenyl substituted with hydroxy, alkoxy, alkyl, trihalomethyl and/or halogen,
wherein at least one of the three phenyl moieties is substituted with halogen or trihalomethyl
or their salts or esters
as COX-1 and/or COX-2 inhibitor for investigating cellular processes in biological in vitro, in vivo or ex vivo systems, preferably cell cultures.
